# FASCICULE DE BREVET EUROPEEN

(11) **EP 2 121 685 B1**
(45) Date de publication et mention de la délivrance du brevet: **17.02.2016**
(21) Numéro de dépôt: 08716957.9
(22) Date de dépôt: 19.02.2008
(51) Int. Cl.: C07D 471/04, C07D 401/12

(54) **NOUVEAUX COMPOSÉS DÉRIVÉS D'INDOLE ET COMPOSITIONS PHARMACEUTIQUES LES CONTENANT**
NEUE INDOLDERIVATVERBINDUNGEN UND PHARMAZEUTISCHE ZUSAMMENSETZUNGEN, DIE DIESE ENTHALTEN
NEW INDOLE DERIVATIVE COMPOUNDS AND PHARMACEUTICAL COMPOSITIONS CONTAINING THE SAME

(30) Priorité: 19.02.2007 FR 0753349; 21.03.2007 FR 0753975
(43) Date de publication de la demande: 25.11.2009
(73) Titulaire: Centre National de la Recherche Scientifique (CNRS), 75016 Paris (FR); Institut Curie, 75005 Paris (FR); Université de Montpellier, 34090 Montpellier (FR)
(72) Inventeur: LEJEUNE, Fabrice, F-59200 Tourcoing (FR); TAZI, Jamal, F-34830 Clapiers (FR); GRIERSON, David, Vancouver BC, V6T 2L1 (CA); RIVALLE, Christian, F-75015 Paris (FR); MAHUTEAU-BETZER, Florence, F-78470 Saint-Rémy-les-Chevreuse (FR)
(74) Mandataire: Le Coupanec, Pascale A.M.P.
(86) Numéro de dépôt international: PCT/EP2008/052025
(87) Numéro de publication internationale: WO 2008/101935

(56) Documents cités:
- WO-A-2005/023255
- VENDOME, JEREMIE ET AL: "Molecular Modeling of Wild-Type and D816V c-Kit Inhibition Based on ATP-Competitive Binding of Ellipticine Derivatives to Tyrosine Kinases" JOURNAL OF MEDICINAL CHEMISTRY , 48(20), 6194-6201 CODEN: JMCMAR; ISSN: 0022-2623, 2005, XP002448769
- CARVALHO, A. C. M. ET AL: "Investigation of geometry and some electronic properties of aza analogues of the ellipticine and olivacine derivatives" THEOCHEM , 539, 273-278 CODEN: THEODJ; ISSN: 0166-1280, 2001, XP002448770
- RIVALLE, C. ET AL: "11H-Pyrido[3',2':4,5]pyrrolo[2,3-g]isoqui nolines (7-azaellipticines) substituted at position 6" TETRAHEDRON , 37(11), 2097-103 CODEN: TETRAB; ISSN: 0040-4020, 1981, XP002448771
- BUU-HOI N P ET AL: "CARCINOGENIC NITROGEN COMPOUNDS. PART LVI. BENZCACRIDINES AND BENZOÅCARBAZOLES METHYLATED ON THE K-ZONE" JOURNAL OF THE CHEMICAL SOCIETY, CHEMICAL SOCIETY, LONDON, GB, 1967, pages 662-665, XP001007988 ISSN: 0368-1769
- TERUHISA TSUCHIMOTO & CO: "Easy access to aryl- and heteroaryl- annulated[a]carbazoles by the indium-catalyzed reaction of 2-arylindoles with propargyl ethers" ANGEW.CHEM. INT. ED., vol. 44, 2005, pages 1336-1340, XP002448772
- OHASHI M ET AL: "ELLIPTICINE AND RELATED ANTICANCER AGENTS" EXPERT OPINION ON THERAPEUTIC PATENTS, ASHLEY PUBLICATIONS, GB, vol. 6, no. 12, 1996, pages 1285-1294, XP008034013 ISSN: 1354-3776
- KANSAL V K ET AL: "THE BIOGENETIC SYNTHETIC AND BIOCHEMICAL ASPECTS OF ELLIPTICINE AN ANTITUMOR ALKALOID" TETRAHEDRON, ELSEVIER SCIENCE PUBLISHERS, AMSTERDAM, NL, vol. 42, no. 9, 1986, pages 2389-2408, XP002293633 ISSN: 0040-4020
- MILLER, R. BRYAN ET AL: "Total synthesis of ellipticine and 9-methoxyellipticine via benzotriazole intermediates" JOURNAL OF ORGANIC CHEMISTRY , 48(6), 886-8 CODEN: JOCEAH; ISSN: 0022-3263, 1983, XP002449133
- MILLER, R. BRYAN ET AL: "A general synthesis of 6H-pyrido[4,3-b]carbazole alkaloids" TETRAHEDRON LETTERS , 21(35), 3319-22 CODEN: TELEAY; ISSN: 0040-4039, 1980, XP002449134
- WOLTHUIS, ENNO ET AL: "Reactions of benzyne with pyrroles" JOURNAL OF ORGANIC CHEMISTRY , 30(1), 190-3 CODEN: JOCEAH; ISSN: 0022-3263, 1965, XP002449135
- DATABASE CA [Online] CHEMICAL ABSTRACTS SERVICE, COLUMBUS, OHIO, US; SCHENKMANM, RONNI L. ET AL: "Structure-activity relationships of mammalian topoisomerase II inhibitors related to ellipticine" XP002449138 extrait de STN Database accession no. 1993:32514 & MEDICINAL CHEMISTRY RESEARCH , 2(3), 165-72 CODEN: MCREEB; ISSN: 1054-2523, 1992,
- DATABASE CA [Online] CHEMICAL ABSTRACTS SERVICE, COLUMBUS, OHIO, US; VERNON, JOHN M. ET AL: "Naphthalen-1,4-imine derivatives with bridgehead substituents" XP002449139 extrait de STN Database accession no. 1977:484708 & JOURNAL OF THE CHEMICAL SOCIETY, PERKIN TRANSACTIONS 1: ORGANIC AND BIO-ORGANIC CHEMISTRY (1972-1999) , (9), 1084-7 CODEN: JCPRB4; ISSN: 0300-922X, 1977,

## Description

Le processus de dégradation des ARNm possédant un codon stop précoce (Nonsense-mediated mRNA decay, NMD) est un processus de contrôle qualitatif identifié dans tous les organismes eucaryotes étudiés à ce jour (CONTI et IZAURRALDE, 2005; MAQUAT, 2004a). Un des rôles de ce mécanisme est de dégrader les ARNm présentant un codon de terminaison prématuré (PTC) de façon à prévenir la synthèse de protéines tronquées qui pourraient ne pas être fonctionnelles ou dont la fonction pourrait être délétère pour la cellule. En outre, la voie NMD a été identifiée comme étant impliquée dans la régulation génique chez la levure, la drosophile et les mammifères (HE *et al.,* 2003 ; MENDELL *et al.,* 2002 ; REHWINKEL *et al.,* 2005 ; SUREAU *et al.,* 2001 ; WOLLERTON *et al,* 2004).

Dans les cellules de mammifères, le NMD prend place après l'épissage des ARN pré-messagers et, dans la plupart des cas, est modulé par un complexe protéique fixé 20 à 24 nucléotides en amont de la jonction exon-exon (Conti and Izaurralde, 2005; Lejeune and Maquat, 2005; Maquat, 2004b). Ce complexe protéique, appelé complexe des jonctions exoniques (exon-junction complex, EJC), est supposé recruter les protéines conservées UPF qui jouent un rôle essentiel, non caractérisé à ce jour, dans le NMD. Durant ce qui est appelé le « premier cycle de traduction » (Ishigaki et al., 2001), les PTCs sont reconnus et les ARNm ciblés sont dégradés par une digestion 5' vers 3' impliquant le retrait de la coiffe et des exoribonucléases telles que hXRN1 et hXRN2/hRAT1, et par une digestion 3' vers 5' impliquant une déadénylation et l'exosome (Chen and Shyu, 2003; Couttet and Grange, 2004; Lejeune et al., 2003).

UPF1 est une phospho-protéine qui subit un cycle de phosphorylation/déphosphorylation durant le NMD (Ohnishi et al., 2003; Page et al., 1999; Pal et al., 2001). Il a été démontré que UPF1 interagit avec des facteurs de terminaison de la traduction chez la levure (Czaplinski et al., 1998) et chez les mammifères (Kashima et al., 2006), et pourrait faire le lien par conséquent, entre l'EJC et le complexe de terminaison de la traduction. Une interaction directe entre hUPF1 et la protéine de liaison à la coiffe CBP80 a été récemment démontré dans les cellules de mammifère (Hosoda et al., 2005), indiquant que hUPF1 établit une interaction complexe avant ou pendant le premier cycle de la traduction. Il a été démontré que la phosphorylation de hUPF1 est effectuée par hSMG1, une kinase apparentée à PI3 (Page et al., 1999; Pal et al., 2001; Yamashita et al., 2001), et requiert la présence de hUPF2 et de hUPF3 (Kashima et al., 2006). Par contraste, la déphosphorylation de hUPF1 requiert la présence d'un complexe multi-protéique composé de hSMG5, hSMG6, hSMG7 et de la protéine phosphatase (PP)-2A (Chiu et al., 2003; Ohnishi et al., 2003). Les protéines hSMG5 et hSMG7 sont localisées principalement dans le cytoplasme dont une fraction est présente dans les « processing bodies » (P-bodies) (Unterholzner and Izaurralde, 2004). hSMG6 est également une protéine cytoplasmique qui se concentre dans certains foyers cytoplasmiques qui semblent distincts des « P-bodies » et dont la nature est toujours indéterminée (Unterholzner and Izaurralde, 2004).

Les « P-bodies » ont été décrits dans les cellules eucaryotes supérieures et inférieures (Cougot et al., 2004; Sheth and Parker, 2003). Chez les mammifères, ces structures cytoplasmiques contiennent de multiples facteurs impliqués dans la dégradation des ARNm incluant des composants de la machinerie de retrait de la coiffe comme DCP1a (Ingelfinger et al., 2002), DCP2 (Ingelfinger et al., 2002; van Dijk et al., 2002), GE1 (Yu et al., 2005) également appelé HEDLS (Fenger-Gron et al., 2005), p54/RCK (Cougot et al., 2004), la déadénylase CCR4 (Cougot et al., 2004), XRN1 (Bashkirov et al., 1997), le complexe LSM1-7 impliqué dans différents aspects des processus liés à l'ARN (Cougot et al., 2004; Ingelfinger et al., 2002), et les composants de la machinerie NMD que sont hUPF1, hSMG5 et hSMG7 (Fukuhara et al., 2005; Unterholzner and Izaurralde, 2004). La fonction des « P-bodies » est toujours indéterminée mais ils pourraient servir de compartiment de stockage pour les ARNm non traduits et pour les protéines impliquées dans la dégradation des ARNm (Brengues et al., 2005; Pillai et al., 2005; Teixeira et al., 2005), et/ou de site de dégradation des ARN (Cougot et al., 2004; Sheth and Parker, 2006).

À l'heure actuelle, près d'un tiers des maladies génétiques humaines auraient pour origine l'apparition d'un codon stop précoce (Frischmeyer and Dietz, 1993 ; Kuzmiak and Maquat, 2006). Dans la grande majorité des cas, ce codon stop entraîne la dégradation de l'ARNm qui le porte par le mécanisme de NMD. Il s'ensuit alors une absence de l'expression du gène concerné et ceci même lorsque la mutation en question permettrait la traduction d'une protéine tronquée fonctionnelle. L'inhibition du mécanisme de NMD chez des patients atteints de telles pathologies devrait permettre de restaurer l'expression de la protéine tronquée fonctionnelle et constitue à ce titre une piste d'intérêt.

Du document WO2005/0232 sont connus des dérivés d'indole pour la préparation d'un médicament utile pour le traitement de maladies liées au processus d'épissage. Ces molécules ont en particulier la capacité d'inhiber les processus d'épissage des ARN pré-messagers.

Les inventeurs ont mis en évidence que des dérivés indoles particuliers étaient capable d'inhiber spécifiquement le NMD *in vitro* et *in vivo,* et que lesdits composés permettaient d'augmenter le niveau d'expression de l'ARNm de la dystrophine présentant des codons stop prématurés dans des lignées cellulaires de patients atteints de la dystrophie musculaire de Duchenne (DMD),

En conséquence, un premier objet de l'invention concerne des composés dérivés d'indole correspondant à la formule II suivante : 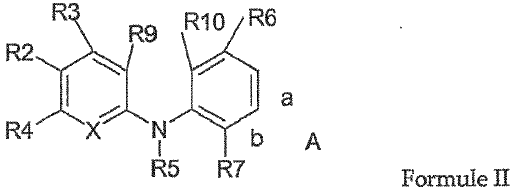 dans laquelle:
- X représente N ou l'anhydro base N⁺R8,
   où R8 représente un atome d'hydrogène, un groupement hydroxyle ou alkyle ou méthoxy éventuellement substitué par un groupement phényle, de préférence R8 représente un atome d'hydrogène,
- R2, R3 et R4 représentent indépendamment un atome d'hydrogène ou un atome d'halogène ou un groupement alkyle en C1-C4
- R5 représente un atome d'hydrogène ou un groupement alkyle, saturé ou insaturé,
- R6 représente un groupement alkyle en C1-C3, de préférence un groupement méthyle ou éthyle et de manière particulièrement préférée R6 représente un groupement méthyle,
- R7 représente un atome d'hydrogène ou un groupement alkyle en C1-C3 éventuellement substitué
- R9 et R10 représentent indépendamment un atome d'hydrogène, un groupement R11, OR11 ou SR11,
   où R11 représente un atome d'hydrogène, un atome d'oxygène, un groupement alkyle en C1-C3 saturé ou insaturé, pouvant contenir un ou plusieurs atomes de soufre, d'oxygène ou d'azote;
- A représente un cycle en position a et qui correspond à 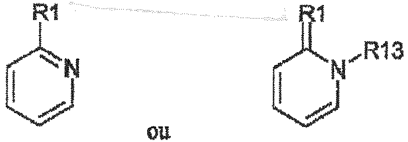 dans lequel :
   R1 représente un atome d'hydrogène, d'oxygène ou d'halogène ou un groupement alkyle ou amine linéaire ou ramifié et/ou insaturé,
   R13 représente un atome d'hydrogène ou un groupement alkyle en C1-C4, et
   des sels pharmaceutiquement acceptables desdits composés.

Par "atome d'halogène", on entend le groupe F, Cl, Br et I, et plus particulièrement le Cl.

Par " groupement alkyle insaturé" , on entend un groupement alkyle présentant au moins une double liaison.

Dans le cas où X représente l'anhydro base N⁺R8 et où R5 représente un hydrogène, il existe un équilibre entre les deux formes suivantes :

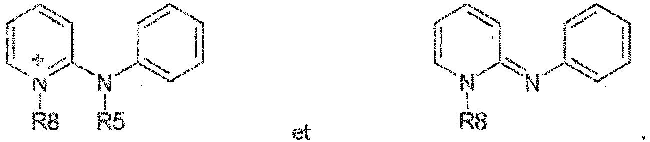

Avantageusement R2, R3 et R4 représentent un atome d'hydrogène.

De préférence, R2, R3 et/ou R4 représentent indépendamment les uns des autres un atome d'halogène choisi dans le groupe comprenant F, Cl, Br et I, de préférence ledit atome d'halogène est un atome de Cl.

Avantageusement, R5 représente un groupement méthyle, éthyle, propyle ou butyle et, de manière particulièrement préférée, R5 représente un atome d'hydrogène.

Avantageusement, R7 représente un groupement méthyle ou éthyle et de manière particulièrement préférée R7 représente un groupement méthyle.

Avantageusement, R1 est un atome d'hydrogène, d'oxygène ou d'halogène.

Dans un premier mode de réalisation particulier, lorsque R9 représente un groupement différent d'un atome hydrogène, alors R10 représente un atome hydrogène.

Avantageusement, R9 représente un groupement OR11, où R11 représente un groupement méthyle.

De préférence, le composé de formule I répond à la formule suivante :

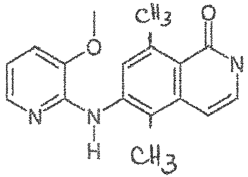

Dans un second mode de réalisation particulier, lorsque R10 représente un groupement différent d'un atome d'hydrogène, alors R9 représente un atome d'hydrogène.

Avantageusement, R9 et R10 représentent tous deux un atome d'hydrogène. Le composé de formule générale II répondant alors à formule Ia suivante : 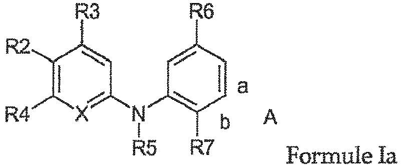 dans laquelle R2, R3, R4, R5, R6, R7, X et le cycle A sont tels que définis précédemment, fait également partie de l'invention.

Un composé préféré de formule Ia est le 5, 8-diméthyl-6(5-méthyl-pyridin-2-ylamino)-isoquinoline.

Un autre composé préféré de formule la est le 5,8-Dimethyl-6-(pyridin-2-ylamino)-2H-isoquinolin-1-one.

De préférence, R9 et R10 représentent un hydrogène et X représente N ou l'anhydro base N⁺R8.

La presente demande décrit également les composés pour lesquels, R9 et R10 représentent ensemble une liaison carbone, le composé de formule générale II répondant alors à la formule Ib suivante : 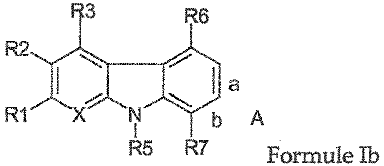 dans laquelle R2, R3, R4, R5, R6, R7, X et le cycle A sont tels que définis précédemment.

Avantageusement, le cycle A représente le groupement : 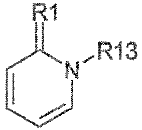 où R1 représente un oxygène, une aminé, ou un groupement alkyle linéaire ou ramifié et/ou insaturé, et R13 représente un groupement alkyle en C1-C4 éventuellement substitué.

Avantageusement encore, le cycle A représente le groupement : 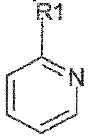 où R1 représente un atome d'hydrogène, d'oxygène ou d'halogène ou un groupement alkyle ou amine linéaire ou ramifié et/ou insaturé, de préférence un atome d'hydrogène ou un halogène, de préférence un atome de chlore.

Dans un autre mode de réalisation préférentiel, A est choisi dans le groupe comprenant :

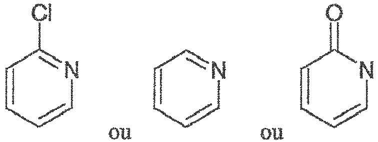

La présent invention décrit également les composés dérivés d'indole de formule II, répondent à la formule I suivante : 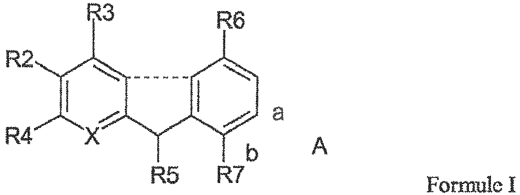 dans laquelle R2, R3, R4, R5, R6, R7, X et le cycle A sont tels que définis précédemment ;
X, R1, R2, R3, R4, R5, R6, R7 et le cycle A sont tels que définis précédemment ;
et/ou des sels pharmaceutiquement acceptables desdits composés.

On entend par la formule I, des composés de formule :

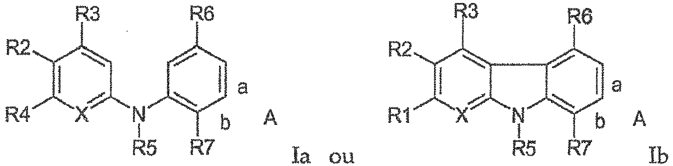

Parmi, les composés dérivés d'indole on cite :
- 6-Chloro-5, 10-dimethyl-11H-pyrido[3',2'] :4,5]pyrrolo[3,2-g]isoquinoline (composé 70) ;
- 5, 10-dimethyl-11H-pyrido[3',2' :4,5]pyrrolo[3,2-g]isoquinoline (composé 71);
- 5, 8-Dimethyl-6-(pyridin-2-ylamino)-2*H*-isoquinolin-1-one; composé 72),
- 8-diméthyl -6-(3-méthoxy-pyridin-2-ylamino)-isoquinolin-1-one (composé 497); et
- 5, 8-diméthyl-6(5-méthyl-pyridin-2-ylamino)-isoquinoline (composé 500).

De préférence, le composé dérivé d'indole est choisi dans le groupe constitué du 5, 8-Dimethyl-6-(pyridin-2-ylamino)-2*H*-isoquinolin-1-one, du 5,8-diméthyl-6-(3-méthoxy-pyridin-2-ylamino)-isoquinolin-1-one et de la 5, 8-diméthyl-6-(5-méthyl-pyridin-2-ylamino)-isoquinoline.

Avantageusement le composé dérivé d'indole est le 5, 8-Dimethyl-6-(pyridin-2-ylamino)-2*H*-isoquinolin-1-one.

Un deuxième objet de l'invention consiste en une composition pharmaceutique comprenant au moins un dérivé d'indole tel que décrit précédemment et, éventuellement, un support pharmaceutiquement acceptable.

A titre d'exemple de support pharmaceutiquement acceptable, la composition peut comprendre des émulsions, des microémulsions, des émulsions huile dans l'eau, des lipides anhydres et des émulsions eau dans l'huile, ou d'autres types d'émulsions.

La composition selon l'invention peut comprendre en outre un ou plusieurs additifs tels que les diluants, les excipients, les stabilisateurs et les conservateurs. De tels additifs sont bien connus de l'homme du métier et sont décrits notamment dans « Ullmann's Encyclopedia of Industrial Chemistry, 6th Ed. » (différents éditeurs, 1989-1998, Marcel Dekker); et dans "Pharmaceutical Dosage Forms and Drug Delivery System "s (ANSEL et al., 1994, WILLIAMS & WILKINS).

La présente invention décrit l'utilisation d'au moins un dérivé d'indole tel que décrit précédemment pour la préparation d'un médicament destiné à traiter, chez un sujet, une maladie génétique résultant d'au moins une mutation entraînant l'apparition d'un codon de terminaison précoce.

Tel qu'utilisé dans la présente demande, le terme « sujet » correspond à un mammifère tel qu'un rongeur, un félin, un canin, un primate ou un humain, de préférence ledit sujet est un humain.

De telles maladies génétiques résultant d'au moins une mutation entraînant l'apparition d'un codon de terminaison précoce sont bien connues et représenteraient près du tiers des maladies génétiques actuellement. À titre d'exemple de telles maladies génétiques, on peut citer la β-thalassémie, le syndrome de Marfan, la dystrophie musculaire de Duchenne (DMD), la dystrophie musculaire de Becker (BMD), la maladie d'Ullrich, le syndrome de Hurler ou la fibrose cystique (CF), plus connue sous le nom de mucoviscidose, et pour des patients présentant un codon de terminaison prématuré dans le gène impliqué dans lesdites maladies génétiques.

De préférence, ladite mutation permet malgré tout d'obtenir une protéine tronquée fonctionnelle.

Les composés selon l'invention ont en effet la capacité d'inhiber le mécanisme de NMD et de permettre la traduction d'ARNm présentant des codons de terminaison prématurés.

Par protéine fonctionnelle, on entend une protéine permettant de restaurer un phénotype sauvage.

Avantageusement, on entend par « protéine fonctionnelle » une protéine présentant une activité suffisante par rapport à la protéine sauvage pour assurer la même fonction que cette dernière et permettant d'obtenir un phénotype sauvage.

Un troisième objet de l'invention consiste en l'utilisation d'un composé dérivé d'indole correspondant à la formule II suivante : 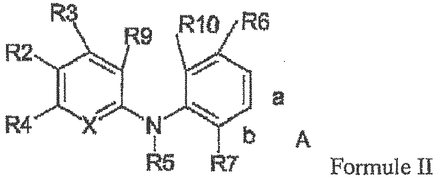 dans laquelle :
- X représente N, CR8 ou l'anhydro base N⁺R8,
   où R8 représente un atome d'hydrogène, un groupement hydroxyle ou alkyle ou méthoxy éventuellement substitué par un groupement phényle, de préférence R8 représente un atome d'hydrogène,
- R2, R3 et R4 représentent indépendamment un atome d'hydrogène ou un atome d'halogène ou un groupement alkyle en C1-C4,
- R5 représente un atome d'hydrogène ou un groupement alkyle, saturé ou insaturé,
- R6 représente un groupement alkyle en C1-C3, de préférence un groupement méthyle ou éthyle et de manière particulièrement préférée R6 représente un groupement méthyle,
- R7 représente un atome d'hydrogène ou un groupement allyle en C1-C3
- R9 et R10 représentent ensemble une liaison carbone ou représentent indépendamment un atome d'hydrogène, un groupement R11, OR11, ou SR11,
   où R11 représente un atome d'hydrogène, un atome d'oxygène, un groupement alkyle en C1-C3 saturé ou insaturé, pouvant contenir un ou plusieurs atomes de soufre, d'oxygène ou d'azote,
- A représente un cycle en position a et qui correspond à 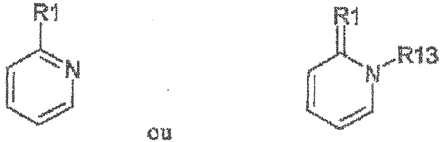 dans lequel :
- R1 représente un atome d'hydrogène, d'oxygène ou d'halogène ou un groupement alkyle ou amine linéaire ou ramifié et/ou insaturé,
- R13 représente un atome d'hydrogène ou un groupement alkyle en C1-C4, et/ou des sels pharmaceutiquement acceptables desdits composés,
pour son utilisation pour traiter, chez un sujet, une maladie génétique choisie dans le groupe comprenant la β-thalassémie, le syndrome de Marfan, la dystrophie musculaire de Duchenne (DMD), la dystrophie musculaire de Becker (BMD), la maladie d'Ulirich, le syndrome de Hurler et la fibrose cystique (CF).

La présente invention concerne également l'utilisation d'un composé de formule II tel que défini précédemment, caractérisée en ce que ledit composé est choisi dans le groupe comprenant :
- 6-Chloro-5, 10-dimethyl-1 1H-pyrido[3',2' :4,5]pyrrolo[3,2-g]isoquinoline ;
- 5,10-dimethyl-11H-pyrido[3',2' :4,5]pyrrolo[3,2-g]isoquinoline ;
- 5,8-Dimethyl-6-(pyridin-2-ylamino)-2H-isoquinolin-1-one;
- 5,8-Dimethyl 6-(3 méthoxy-pyridin-2-ylamino)-isoquinolin-1-one ; et
- 5 ,8-diméthyl-6-(5-méthyl-pyridin-2-ylamino)-isoquinoline.

L'invention décrit également une méthode de traitement thérapeutique d'un sujet pour une maladie génétique résultant d'au moins une mutation entraînant l'apparition d'un codon de terminaison précoce comprenant l'administration d'une quantité thérapeutiquement efficace d'une composition pharmaceutique telle que décrite précédemment.

Par « quantité thérapeutiquement efficace », on entend une quantité permettant d'induire l'inhibition du NMD. L'homme du métier sera à même de déterminer ladite quantité thérapeutiquement efficace au regard de ses connaissances générales et des méthodes décrites dans les exemples.

Les composés pourront être administrés par tout mode d'administration comme par exemple par voie intramusculaire, intraveineuse, orale, etc.

Les exemples qui suivent sont fournis à titre d'illustration et ne sauraient limiter la portée de la présente invention.

### Exemples :

### I-MATERIEL ET METHODES

### I-1 Composés indoles

Tous les composés indoles polycycliques utilisés étaient suspendus dans du DMSO à 20 mg/ml, puis préparé à une dilution 5 mM dans10% DMSO (v/v).

Les composés testés sont présentés dans le tableau I suivant :

**Tableau I**

| Composés | Formule | Nomenclature |
|---|---|---|
| 13 hors invention | 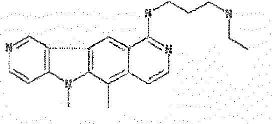 | N-(5,6-Dimethyl-5H-pyrido[3',4':4,5 ]pyrrolo[2,3-g]isoquinolin-10-yl)-N '-ethyl-propane-1,3-diamine |
| 15 hors invention | 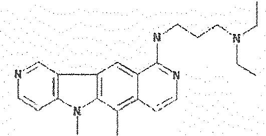 | N'-(5,6-Dimethyl-5H-pyrido[3',4':4, 5]pyrrolo[2,3-g]isoquinoiin-10-yl)-N,N-diethyl-propane-1,3-diamine |
| 17 hors invention | 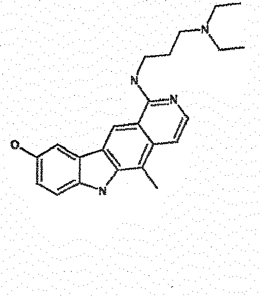 | 1-(3-Diethylamino-propylamino)-5-me thyl-6H-pyrido[4,3-b]carbazol-9-ol |
| 29 hors invention | 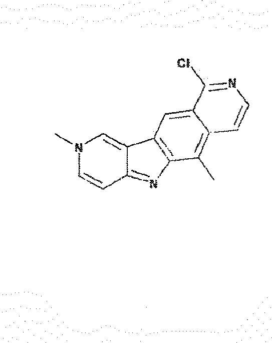 | 10-Chloro-2,6-dimethyl-2H-pyrido[3' ,4':4,5]pyrrolo[2,3-g]isoquinoline |
| 35 hors invention | 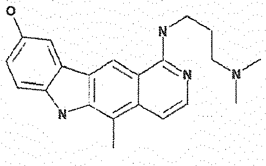 | 1-(3-Dimethylamino-propylamino)-5-m ethyl-6H-pyrido[4,3-b]carbazol-9-ol |
| hors invention 37 | 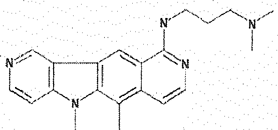 | N'-(5,6-Dimethyl-5H-pyrido[3',4':4, 5]pyrrolo[2,3-g]isoquinolin-10-yl)-N,N-dimethyl-propane-1,3-diamine |
| 39 hors invention | 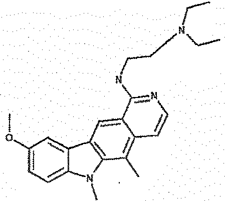 | N,N-Diethyl-N'-(9-methoxy-5,6-dimet hyl-6H-pyrido[4,3-b]carbazol-1-yl)-ethane-1,2-diamine |
| 67 hors invention | 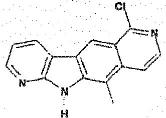 | 6-Chloro-10-melhyl-11H-pyrido[3',2' :4,5]pyrrolo[3,2-g]jisoqulnoline |
| 70 invention | 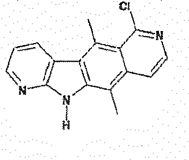 | 6-Chioro-5,10-dimethyl-11H-pyrido[3 ',2':4,5]pyrrolo[3,2-g]isoquinoline |
| 71 invention | 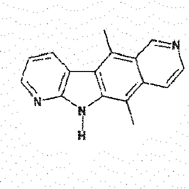 | 5, 10-dimethyl-11H-pyrido[3',2':4,5]pyrrolo[3,2-g]isoquinoline |
| 72 invention | 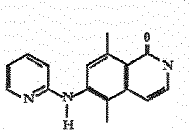 | 5,8-Dimethyl-6-(pyridin-2-ylamino)-2*H*-isoquinolin-1-one |
| 81 hors invention | 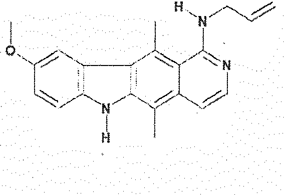 | Allyl-(9-methoxy-5,11-dimethyl-6H-p yrido[4,3-b]carbazol-1-yl)-amine |
| 497 invention | 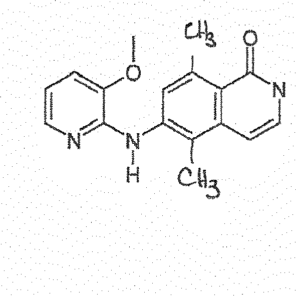 | 5, 8-diméthyl -6-(3 méthoxy-pyridin-2-ylamino)-isoquinolin-1-one |
| 500 invention | 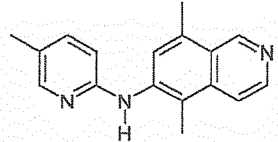 | 5,8-diméthyl-6-(5 méthyl-pyridin-2-ylamino)-isoquinoline |

### 1-3 Synthèse des composés selon la présente invention

La synthèse des composés 17, 35 et 39 est décrite dans la publication suivante : RIVALLE et al., J. Med. Chem. 1983, 26, 181-185.

Le composé 81 est obtenu par la même procédure générale que les composés 17, 35 et 39, à partir du composé décrit comme 8a dans la publication et de l'allylamine.

La synthèse des composés 67, 70 et 71 est décrite dans RIVALLE et al., Tetrahedron 1981; 37, 2097-2103

La synthèse des composés 13, 15 et 37 est décrite dans BISAGNI et al. Heterocycles 1988, 27, 1671-1678.

Le composé 29 est obtenu par le procédé de synthèse suivant: le composé décrit la dans la publication de RIVALLE et al.,( J. Med. Chem. 1983, 26, 181-185) est mis en suspension dans du DMF. Un excès d'iodure de méthyle (10 equiv) est ajouté. Le milieu réactionnel est chauffé à 80°C pendant 1 h puis refroidit à température ambiante. Une solution de Na₂CO₃ 2M est ajoutée et le milieu réactionnel est porté à 80°C pendant 18h. Le produit est extrait par le dichlorométhane.

Les composés 72, 497 et 500 sont obtenus par le procédé de synthèse suivant : La bromopyridine (1 équivalent) et l'amine (1 équivalent) adéquates sont placées dans du *tert*-butanol en présence de 2mol% de Pd(Oac)₂ de 3.5mol% de Xantphos et de 2.4 équivalents de Cs₂CO₃. Le milieu réactionnel est porté à 90°C pendant 48h puis il est filtré sur célite puis purifié sur colonne de silice (CH₂CL₂/ EtOH 95 :5).

Pour la synthèse du composé 72, on utilise de la 2-bromopyridine et de la 6-amino-5,8-diméthyl-2H-isoquinolin-1-one comme amine. Cette amine est décrite dans DUCROCQ et al. (Tetrahedron 1979, 35, 142)

Pour la synthèse du composé 497 on utilise de la 2-bromo-3-méthoxypyridine et de la 6-amino-5,8-diméthyl-2H-isoquinolin-1-one comme amine.

Pour la synthèse du composé 500, on utilise de la 2-bromo-5-méthylpyridine et et de la 5,8-diméthyl-isoquinolin-6-ylamine comme amine. Cette amine est décrite dans BALKAN et al. (Australian Journal of Chemistry 1969, 22, 2489).

### I-3 Constructions

Les constructions β-globine Norm et Ter ont été obtenues par amplification par PCR des constructions β-globine WT et NS39 (Thermann et al., 1998) en utilisant l'amorce sens 5'GCAACCTCAAGCTTACACCATGGTGCACCTGAC3' (SEQ ID NO:1) et l'amorce antisens 5'AGAAAGCAGATCTGCTTAGTGATACTTGTG3' (SEQ ID NO:2). Les fragments amplifiés ont été clonés aux positions HindIII/BglII d'un plasmide pRSVbgal modifié comprenant 24 sites MS2 (Fusco et al., 2003).

### I-4 Mesure NMD par RT-PCR

Les cellules HeLa ont été cultivées dans des boîtes de 60 mm dans du milieu DMEM (Dulbecco's Modified Eagle Medium, GIBCO-BRL) supplémenté avec 10% (v/v) de sérum de veau foetal à 37°C et 5% CO₂ Les cellules (1 x 10⁶) ont alors été transfectées avec 3µg de plasmides tests pmCMV-Gl (Norm ou 39Ter)(Sun et al., 1998) ou 3µg de plasmides tests pmCMV-GPx1 (Norm ou 46Ter)(Moriarty et al., 1998) et de 1µg de plasmide de référence phCMV-MUP (Belgrader and Maquat, 1994) en utilisant le kit LIPOFECTAMINE PLUS REAGENT (INVITROGEN) selon les instructions du fabricant. 24 heures après transfection, les cellules ont été traitées pendant 20 heures avec 5 µM de chacun des composés à tester ou avec du DMSO 0.01% (v/v) en tant que contrôle. L'ARN total a été purifié en utilisant du TRI Reagent (SIGMA-ALDRICH) selon les instructions du fabricant, puis une transcription inverse des ARNm Gl, GPx1 et MUP a été effectuée avant une amplification par PCR en présence de nucléotide dCTP radiomarqué au ³²P. Les conditions de PCR et la méthode d'analyse ont été décrites précédemment (Ishigaki et al., 2001). Les produits de PCR ont été quantifiés sur Typhoon 9200 (Amersham Biosciences).

### I-5 Test d'efficacité de traduction

Les cellules HeLa ont été transfectées avec 2 µg de plasmide pFluc et 1 µg de plasmide pRluc. 24 heures après transfection, les cellules ont été incubées en présence de DMSO 0.01% (v/v), ou de différents composés à tester (5 µM) pendant 20 heures ou de 100 µg/ml de cycloheximide pendant 4 heures avant récolte des cellules. L'activité luciférase a été quantifiée sur l'équivalent de 2x10⁵ cellules sur un microLumat LB 96P (EG&G Berthold) en utilisant le kit Dual Glo Luciferase (Promega) selon les instructions du fabricant. L'activité luciférase a ensuite été normalisée au regard du niveau d'ARNm Fluc et Rluc.

### I-6 Activité luciférase pour mesurer l'intégrité de la voie de dégradation siRNA en présence des composés inhibiteurs du NMD

Les cellules ont été cultivées dans des plaques 6 puits et transfectées en utilisant le kit Lipofectamine Plus reagent (Invitrogen) avec 50ng d'ARN rapporteur RLperfect (Pillai et al., 2005), 200ng de plasmide pG13 codant pour la '*Firefly luciferase'* et 4µg de plasmide pTzU6. 24 heures après transfection, les composés à tester ont été ajoutés aux puits correspondants. 48 heures après transfection, l'activité luciférase a été mesurée avec le kit Dual Glo Luciferase (Promega) selon les instructions du fabricant.

### I-7 Mesure du niveau de phosphorylation de FLAG-hUPF 1 par analyse de tel 2D

Des cellules 293T (10⁶) ont été transfectées avec 1µg de plasmide *pFLAG-hUpf1* (Sun et al., 1998) en utilisant le kit Lipofectamine Plus Reagent (Invitrogen) selon les instructions du fabricant. Après 12 heures, le sérum a été retiré du milieu de culture pendant 24 heures avant d'ajouter 5 µM de composés à tester ou, en tant que contrôle, de DMSO 10% (v/v) pendant 3 heures à 37°C et 5% CO₂ Alors, 10% de sérum est ajouté de nouveau pendant 1 heure à 37°C et 5% CO₂ Les protéines totales ont été purifiées avec un tampon de lyse comprenant 8M urée, 2% CHAPS et 40mM Tris base. La migration dans la première dimension a été effectuée selon le protocole décrit par AMERSHAM-BIOSCIENCES pour l'électrophorèse en 2D avec un pH Gradient immobilisé. De l'Immobiline^{™} DryStrip pH 3-10 (18 cm) a été utilisée pour séparer les protéines selon leur point isoélectrique. Alors, la seconde dimension a été effectuée en chargeant la première dimension sur un gel SDS-PAGE 10%. Finalement, les protéines ont été transférées sur une membrane de nitrocellulose avant leur incubation avec un anticorps anti-FLAG (SIGMA-ALDRICH) dans du TBS contenant 0.05% TWEEN une nuit à 4°C, suivie d'une incubation avec un anticorps de chèvre anti-souris conjugué à la peroxydase (PIERCE). Les protéines sont alors détectées en utilisant le substrat « SuperSignal West Femto Maximum Sensitivity Substrate » (PIERCE).

### I-8 Détermination de l'étape de blocage du NMD

Cette expérience a été réalisée comme décrit précédemment (Hosoda et al., 2005).

### I-9 Immunofluorescence, test FISH et analyse d'image

Des cellules HeLa ont été cultivées sur des lames de verre de 12 mm dans du DMEM 10% (v/v) FBS. Les cellules (10⁵) ont été transfectées de façon transitoire avec 500 ng de plasmides pGFP-GE1 (Yu et al., 2005), pYFP-hSmg5, pYFP-hSmg6, YFP-hSmg7 (Unterholzner and Izaurralde, 2004), pGFP-CCR4, pCFP-DCP1la (Cougot et al., 2004), pCI-neo-FLAG-hUpf1 (Sun et al., 1998), pcDNA3-hUpf3a-FLAG, pcDNA3-hUpf3b-FLAG (Lykke-Andersen et al., 2000), pmCMV-Gl (Norm ou 39Ter) (Sun et al., 1998) ou pmCMV-GPx1 (Norm ou 46Ter) (Moriarty et al., 1998). 24 heures après transfection, les cellules ont été traitées avec 5µM de composés à tester ou de DMSO 0,001% (v/v) en tant que contrôle. Après 20 heures, les cellules ont été fixées en utilisant une solution de formaline (SIGMA-ALDRICH) pendant 10 min à température ambiante et perméabilisées avec une solution d'éthanol à 70% sur la nuit à 4°C.

Pour les tests d'immunofluorescence, les cellules fixées ont été incubées avec un anticorps de souris anti-FLAG (SIGMA-ALDRICH) pendant 2 heures à température ambiante, lavées trois fois dans du PBS puis incubées avec un anticorps de souris conjugué au Cy3 ou au FITC (JACKSON IMMUNORESEARCH) pendant une heure à température ambiante. Finalement, les cellules ont été lavées trois fois dans du PBS avant d'être incubées dans du Hoechst (2 ng/µl) (SIGMA-ALDRICH) pendant 2 minutes à température ambiante.

Pour les expériences de FISH, les cellules fixées ont été incubées dans un tampon de pré-hybridation (ARNt 125 µg/ml, ADN de hareng 500 µg/ml, BSA 1 mg/ml, dextran sulfate 0.1 g/ml, 50% formamide, 2X tampon SSC) à 37°C pendant 1 heure dans un incubateur de culture de tissus. Puis, les cellules fixées sont incubées sur la nuit dans un incubateur de culture de tissus avec le tampon d'hybridation (tampon de préhybridation avec des sondes marquées au Cy3), lavées trois fois dans un tampon 2X SSC à 37°C, trois fois dans du tampon 1X SSC à température ambiante et finalement incubées avec du Hoechst (2ng/µl) (SIGMA-ALDRICH) pendant deux minutes à température ambiante. La sonde 5'CGATCTGCGTTCTACGGTGGT3' (SEQ ID NO :3) marquée au Cy3 aux extrémités 5' et 3' a été utilisée pour détecter les ARNm G1 Ter ou GPX1 Ter.

Les cellules fixées ont été observées avec un microscope DMRA (Leica), un objectif huile PL APO 63x (NA 1.32) avec des filtres A4 (pour hoechst), GFP et Y3 (for Cy3).

### I-10 Immunopurification et analyse par western blot

L'immunopurification de hUPF1 et l'analyse par western blot ont été effectuées selon un protocole décrit précédemment (Lejeune and Maquat, 2004) en utilisant un anticorps anti hUPF1 (Ohnishi et al., 2003). Les analyses par western blot ont été réalisées en utilisant une dilution au 1/250 d'anticorps de lapin anti hUPF1, anti-hSMG5, anti-hSMG6 ou anti-hSMG7 (Ohnishi et al., 2003), une dilution au 1/1000 d'anticorps de lapin anti-hUPF3/3X (Ishigaki et al., 2001) ou d'anticorps de souris anti-TUBULIN (SIGMA-ALDRICH). Les protéines ont été détectées en utilisant le substrat «SuperSignal West Pico Chemiluminescent Substrate » ou « SuperSignal West Femto Maximum Sensitivity Substrate » (PIERCE).

### II-RESULTATS

### II-1 Identification de nouveaux inhibiteurs du NMD :

Des cellules Hela ont été transfectées par deux plasmides tests codant pour les ARN de la β-globine (Gl) et de la glutathione peroxidase 1 (GPx1), et présentant un codon de terminaison prématuré (Ter) ou non (Norm). L'ARNm Gl est sujet à un NMD associé au noyau dans les cellules non-érythroïdes (Thermann et al., 1998; Zhang et al., 1998) alors que l'ARNm GPx1 est sujet à un NMD cytoplasmique (Moriarty et al., 1998). En outre, un plasmide de référence codant pour l'ARNm d'une protéine urinaire majeure (MUP) a également été introduit dans les cellules (Ishigaki et al., 2001).

24 heures après transfection, les cellules ont été incubées pendant 20 heures avec du DMSO(-) en tant que contrôle, ou avec 5 µM des composés indoles présentés dans le tableau I. Après 20 heures, les ARN totaux ont finalement été purifiés et analysés par RT-PCR.

Les résultats pour les différents composés testés sont résumés dans le tableau II.

**Tableau II**

| Composés | Stabilisation de l'ARNm G1 Ter | Stabilisation de l'ARNm GPx1 Ter |
|---|---|---|
| 13 | - | - |
| 15 | - | - |
| 17 | - | - |
| 29 | - | - |
| 35 | - | - |
| 37 | - | - |
| 39 | - | - |
| 70 | +++ | ++ |
| 71 | +++ | ++ |
| 72 | +++ | ++ |
| 81 | - | - |
| 497 | +++ | ++ |
| 500 | +++ | ++ |

Les résultats ont montrés que, parmi les 30 composés testés, cinq d'entre eux permettent de stabiliser aussi bien le niveau d'ARNm G1 Ter que de GPx1 Ter et ceci de façon dose dépendante. Ce résultat a pu en outre être confirmé par la technique de RPA. Ces résultats permettent de conclure que les composés 70, 71, 72, 497 et 500 constituent des inhibiteurs du NMD et ceci aussi bien au niveau nucléaire que cytoplasmique. À noter que le niveau d'inhibition observé pour ces composés était similaire à ceux obtenus avec d'autres inhibiteurs du NMD tels que la cycloheximide ou l'inactivation par siRNA de l'expression des gènes codant pour hDCP2 ou hPARN (Ishigaki et al., 2001; Lejeune et al., 2003) et que ces composés ne montraient aucune toxicité cellulaire dans les conditions expérimentales (concentration maximale testée de 125 µM).

À ce niveau, la spécificité des composés identifiés a été testée dans différents systèmes (absence d'inhibition de l'épissage des introns, absence de l'inhibition de la traduction et absence de formation de granules de stress) et a permis de mettre en évidence que ces composés constituent de nouveaux inhibiteurs spécifiques du NMD.

### II-2 Inhibition du NMD en amont de hUPF1:

Dans le but d'identifier le mode d'inhibition des composés identifiés, lesdits composés ont été testés sur un système mimant le recrutement séquentiel des facteurs du NMD sur un ARNm (Kim et al., 2005; Lykke-Andersen et al., 2000). Pour se faire, des cellules ont été transfectées avec deux types de constructions dont l'une codait pour l'ARNm de la *Firefly Luciferase* (*Fluc*) mRNA contenant 8 sites de liaison pour la protéine MS2 sur son 3'UTR, et la seconde codait soit pour la protéine MS2, soit pour l'une des protéines de fusion suivante : MS2-hUPF1, MS2-hUPF2 or MS2-hUPF3X. En outre, les cellules HeLa ont également été transfectées avec une construction codant pour l'ARNm de la *Renilla Luciferase* (*Rluc*) de façon à normaliser le niveau d'ARN analysé.

Les cellules ont été ensuite incubées pendant 20 heures avec le composé 70 ou du DMSO (-) en tant que contrôle négatif. Le niveau des ARNm *Rluc* et *Fluc* a alors été mesuré comme décrit précédemment (Hosoda et al., 2005). À titre de contrôle, l'expression de chacune des protéines de fusion MS2 a été déterminée par western blot.

Les résultats ont montré que dans chaque cas, le composé 70 n'affectait pas le niveau d'expression des protéines de fusion MS2, lequel n'était jamais supérieur au niveau d'expression des protéines endogènes. Comme attendu et en présence de DMSO, le niveau d'expression des ARNm *FLuc* était inférieur dans les cellules exprimant l'une des protéines de fusion MS2-hUPF fusion par rapport aux cellules exprimant uniquement MS2. En revanche, les résultats ont révélé que le composé 70 interfère avec la dégradation induite par MS2-hUPF2 ou MS2-hUPF3X, mais n'a pas d'effet sur la dégradation induite par MS2-hUPF1. À noter que le rapport des ARNm *FLuc*/*RLuc* et le niveau d'inhibition induit par le composé 70 sont très similaire à ceux observés en réponse à une inactivation par siRNA de l'expression du gène codant pour hCBP80 (Hosoda et al., 2005).

Finalement, les résultats indiquent que l'inhibition du NMD par le composé 70 en aval du recrutement de hUPF3X ou de hUPF2 et en amont des fonctions de hUPF1.

### II-3 Le composé 70 ne prévient pas l'interaction entre hUPF1 et hUPF3X :

Au regard des résultats précédents, il a été envisagé que le composé 70 pouvait prévenir le recrutement de hUPF1 à l' EJC via son interaction avec d'autres protéines hUPF. Dans ce but, la protéine hUPF1 a été immunoprécipitée à partir d'extraits de cellules HeLa dans des conditions préservant l'intégrité des mRNPs (Lejeune and Maquat, 2004).

Le composé 70 ou du DMSO (-) a été ajouté aux cultures de cellules 20 heures avant l'immunoprécipitation (IP). Dans la mesure où il a été démontré que la protéine hUPF2 n'était pas essentielle dans certains cas de NMD (Gehring et al., 2005), l'analyse s'est focalisée sur la présence de la protéine hUPF3X dans chaque immunoprécipitation. Comme contrôle de spécificité, il a été démontré l'absence de protéine tubuline dans chacune des immunoprécipitations et une immunoprécipitation non spécifique a été réalisée avec du sérum de lapin normal dans laquelle aucune protéine n'a été détectée.

Les résultats ont montré que la protéine hUPF3X était présente dans les immunoprécipitations de hUPF1 même lorsque les cellules étaient préalablement incubées avec le composé 70.

En conséquence, l'interaction entre hUPF1 et hUPF3X n'est pas abolit par le composé 70 ce qui suggère que ce composé n'inhibe pas le recrutement de hUPF1 à l'EJC.

### II-4 Le composé 70 stabilise les formes hyperphosphorylées de hUPF1:

Dans la mesure où hUPF1 requiert un cycle de phosphorylation et de déphosphorylation durant le NMD (Ohnishi et al., 2003), le niveau de phosphorylation en réponse à la présence du composé 70 a été testée. À cette fin, le niveau de phosphorylation de hUPF1 a été testé dans des cellules incubées avec le composé 70 ou avec du DMSO (-) par une analyse en gel 2D. Dans la mesure où la phosphorylation de hUPF1 est influencée par le sérum (Pal et al., 2001), des cellules 293T plutôt que HeLa ont été utilisé en raison de leur capacité à bloquer leur division cellulaire à la même étape du cycle cellulaire en l'absence de sérum. Les cellules ont été transfectées par le vecteur d'expression pCI-neo-FLAG-hUpfl (Sun et al., 1998), synchronisées par absence de sérum 12 heures après transfection pendant 24 heures. Finalement, du DMSO ou 5 µM du composé 70 ont été ajoutés pendant 3 heures avant d'ajouter du sérum pendant une heure.

Les résultats ont montré que sans ajout de sérum, la protéine FLAG-hUPF1 migre en ne formant qu'un seul point correspondant à la protéine non phosphorylée (Pal et al., 2001). Après l'addition de sérum, on observe une phosphorylation intermédiaire de la protéine FLAG-hUPF1 lorsque les cellules ont été incubées avec du DMSO et la stabilisation d'isoformes phosphorylées supplémentaires de FLAG-hUPF1 lorsque les cellules ont été incubées avec le composé 70.

En conséquence, le composé 70 permet donc de stabiliser des isoformes hyperphosphorylées de la protéine hUPF1.

Dans la mesure où il a été proposé que la protéine hUPF1 se localiserait au niveau des « P-bodies » lorsqu'elle est hyperphosphorylée (Unterholzner and Izaurralde, 2004), la localisation cellulaire de la protéine FLAG-hUPF1 dans des cellules HeLa en présence et en l'absence du composé 70 a été testée.

Les résultats montrent que la protéine hUPF1 exogène est distribuée de façon égale dans le cytoplasme lorsque les cellules ont été incubées avec le DMSO comme cela a été démontré précédemment pour des cellules non traitées (Mendell et al., 2002), mis à part dans des expériences de co-expression avec la protéine hSMG7, laquelle induit le recrutement de la protéine hUPF1 au niveau des « P-bodies » (Unterholzner and Izaurralde, 2004). En revanche, et lorsque les cellules ont été préalablement traitées avec le composé 70, on observe des concentrations cytoplasmiques de FLAG-hUPF1 dans des structures qui colocalisent avec GFP-GE1, YFP-hSMG7 ou CFP-hDCP1a, lesquelles constituent des marqueurs communs des « P-bodies ».

En conséquence, le composé 70 induit l'accumulation des isoformes hyperphosphorylées de hUPF1 au niveau des « P-bodies » soit par la stimulation de la phosphorylation soit par l'inhibition de la déphosphorylation.

De manière à déterminer lequel de ces deux mécanismes serait induit par les composés identifiés, une analyse complémentaire des immunoprécipitats de la protéine hUPF1 à partir de cellules Hela incubées ou non en présence de composé 70 a été effectuée.

Dans un premier temps, l'analyse de hUPF1 avec son complexe de déphosphorylation a été effectuée. Les résultats ont montré que hSMG5, hSMG6 et hSMG7 peuvent être détectés dans les cellules traitées avec le DMSO mais que hSMG5 était en revanche indétectable lorsque les cellules HeLa avaient été préalablement incubées avec le composé 70.

En conséquence, les résultats montrent que le composé 70 déstabilise l'interaction entre hUPF1 et hSMG5. Finalement, la présence de hSMG1 et de hUPF3X dans les immunoprécipitations effectuées sur les cellules incubées ou non avec le composé 70 suggère fortement que les composés identifiés n'ont pas d'influence sur l'interaction entre hUPF1 et son complexe de phosphorylation (Fig. 3A). Les résultats obtenus plaident pour le fait que l'état d'hyperphosphorylation de hUPF1 observé serait lié à un défaut de déphosphorylation, en raison de l'absence d'interaction entre hUPF1 et hSMG5, plutôt qu'à une activation de la phosphorylation. Cette conclusion est finalement en accord avec l'identification de hSMG5 en tant que composant essentiel pour la déphosphorylation de hUPF1 (Ohnishi et al., 2003).

### II-5 hSMG5 est exclut des « P-bodies » en présence du composé 70:

Il est connu que hSMG5 et hSMG7 sont localisés dans le cytoplasme et plus spécifiquement au niveau des « P-bodies » (Unterholzner and Izaurralde, 2004).

Des cellules HeLa ont été transfectées avec les vecteurs d'expression codant pour YFP-hSMG5, YFP-hSMG6 ou YFP-hSMG7 (Unterholzner and Izaurralde, 2004) et CFP-hDCP1a en tant que marqueur des « P-bodies ». Après 24 heures, les cellules ont été incubées en présence de DMSO ou de 5 µM de composé 70.

Comme cela avait été démontré précédemment, en l'absence d'inhibiteurs, YFP-hSMG5, YFP-hSMG6 et YFP-hSMG7 sont concentrés dans des foci cytoplasmiques (Fukuhara et al., 2005; Unterholzner and Izaurralde, 2004), lesquels foci ont été identifiés comme étant majoritairement des « P-bodies » lorsque CFP-DCP1a est utilisé en tant que marqueur des « P-bodies ».

En présence du composé 70, les foci cytoplasmiques contenant YFP-hSMG6 ou YFP-hSMG7 colocalisent avec le marqueur des «P-bodies» CFP-DCP1a. En revanche, hSMG5 n'est plus observé dans des foci cytoplasmiques, mais présente une large distribution cytoplasmique dans les cellules traitées avec le composé 70.

Dans la mesure où il a été démontré que le composé 70 inhibe le NMD et induit la concentration de hUPF1 dans les P-bodies comme démontré avec la protéine hUPF1 exogène ou endogène, la localisation des trois protéines hSMG endogènes au niveau de foci cytoplasmiques a été testée. Cette localisation n'avait jamais été observée du fait d'une expression trop faible de ces protéines. En traitant les cellules avec le composé 70, il était envisageable d'obtenir une stabilisation de ces facteurs au sein des P-bodies.

Les résultats ont montré que les protéines endogènes ne sont pas détectées au niveau de foci cytoplasmique pour les cellules traitées avec le DMSO. En revanche, et lorsque les cellules sont incubées avec le composé 70, les résultats ont montré une concentration des protéines hSMG6 et hSMG7 au niveau des « P-bodies » alors que hSMG5 n'est pas détectée au niveau de foci cytoplasmiques confirmant les résultats obtenus avec les protéines exogènes.

Finalement, les résultats montrent que le composé 70 modifie la localisation cellulaire de hSMG5 en l'excluant des « P-bodies ». Cette observation est consistante avec la perte d'interaction entre UPF1 et SMG5 lorsque les cellules sont incubées avec le composé 70.

### II-6 hUPF3 et hUPF3X localisent au niveau des « P-bodies » lorsque le NMD est bloqué par le composé 70

Puisque certains facteurs du NMD, comme hUPF1, hSMG5, hSMG6 ou hSMG7 se localisent au niveau des « P-bodies » (Unterholzner and Izaurralde, 2004), il a été envisagé que d'autres facteurs du NMD puissent passer dans les « P-bodies » de façon transitoire au moins. Comme le composé 70 bloque le NMD à une étape à laquelle hUPF1 est confinée au niveau des « P-bodies », la localisation cellulaire de hUPF3 et de hUPF3X dans les cellules traitées et non traitées a été testée. Il a été démontré que ces deux protéines sont principalement des protéines nucléaires dans les cellules non traitées (Serin et al., 2001).

Des cellules HeLa ont été transfectées avec des vecteurs d'expression codant pour hUPF3-FLAG ou hUPF3X-FLAG, et avec l'un des marqueurs de « P-bodies » suivant : YFP-hSMG6, YFP-hSMG7, GFP-GE1 et CFP-hDCP1a. Les cellules ont été traitées avec du DMSO ou le composé 70 avant de réaliser des expériences d'immunofluorescence. Comme pour les cellules non traitées (Serin et al., 2001), hUPF3 ou hUPF3X se localisent essentiellement au niveau du noyau lorsque les cellules sont incubées avec du DMSO (-). En revanche, et après incubation des cellules en présence du composé 70, les résultats ont montré une localisation cytoplasmique de hUPF3 et de hUPF3X avec des accumulations dans des foci, correspondant à des « P-bodies ».

### II-7 les ARNm contenant des PTC s'accumulent dans les « P-bodies » en présence du composé 70:

Dans la mesure où les facteurs du NMD s'accumulent au niveau des « P-bodies » en présence du composé 70, la localisation des substrats du NMD a été étudiée. Chez la levure, il a en effet récemment été mis en évidence que lorsque les facteurs du NMD s'accumulaient dans les « P-bodies », les ARNm contenant des PTC s'accumulaient également dans les « P-bodies » lorsque le NMD était bloqué (Sheth and Parker, 2006).

Des cellules HeLa ont été transfectées avec les vecteurs pmCMV-Gl Ter ou pmCMV-GPx1 Ter, et la localisation des ARNm résultant a été analysée avec les marqueurs de « P-bodies » suivants : GFP-GE1, YFP-hSMG6, YFP-hSMG7, CFP-hDCP1a, GFP-hCCR4, FLAG-hUPF1, hUPF3-FLAG et hUPF3X-FLAG.

Les résultats ont montré qu'en l'absence d'inhibiteurs, aucun ARNm contenant des PTC n'a pu être détecté, probablement du fait de leur dégradation rapide par le NMD. En revanche, et après traitement avec le composé 70, les ARNm contenant des PTC sont stabilisés et détectés essentiellement au niveau d'agrégats cytoplasmiques colocalisant avec chacune des protéines de fusion de hUPF testées.

Finalement, les résultats ont montré que les ARNm contenant des PTC étaient présent dans les « P-bodies » ou adjacents à ces derniers lorsque le NMD était inhibé par le composé 70.

L'accumulation des ARNm contenant des PTC au niveau des « P-bodies » lorsque le NMD est bloqué dans des cellules de mammifère a également été confirmée par une approche plus résolutive. Dans cette dernière, les ARNm sont marqués avec 24 répétitions MS2 ce qui permet la détection de molécules d'ARNm uniques par hybridation *in situ* (Fusco et al., 2003).

Les résultats ont montré que, dans les cellules contrôles, les ARNm contenant des PTC sont détectés essentiellement au niveau du noyau et les molécules cytoplasmiques détectées ne s'accumulent pas au niveau des « P-bodies ». Lorsque le NMD est inhibé par le compsé 70, les résultats ont montré une accumulation des ARNm contenant des PTC au niveau du cytoplasme et, plus spécifiquement, dans des structures colocalisant avec les « P-bodies ».

En conséquence, ces résultats confirment que les ARNm sujet au NMD s'accumulent au niveau des « P-bodies » lorsque leur dégradation est inhibée et cette conclusion ne semble pas être cellule-spécifique.

Finalement, les résultats ont montré qu'aucun ARNm sauvage n'est détecté dans les « P-bodies » après incubation avec le composé 70, ce qui conforte une fonction d'inhibiteur spécifique du NMD des composés identifiés plutôt qu'une fonction générale d'inhibition de la dégradation des ARNm.

### II-8 Stabilisation d'ARNm de dystrophine présentant un codon stop prématuré:

Le gène de la dystrophine est composé de 79 exons, dont les exons 70 à 79 codent pour une partie de la protéine qui n'est pas essentielle pour la fonction de cette protéine dans le muscle.

Parmi les patients atteints de la dystrophie musculaire de Duchenne (DMD), il existe des patients qui portent une mutation non sens dans l'un des exons 70 à 79. Pour autant, ces patients n'expriment pas la protéine dystrophine du fait de la dégradation de l'ARNm par le NMD et ceci bien que la protéine tronquée soit fonctionnelle. En conséquence, une inhibition du NMD chez ces patients permettrait de synthétiser cette protéine tronquée mais fonctionnelle.

Deux lignées cellulaires issues de patients atteints de DMD du fait respectivement d'une rétention de l'intron 70 qui apporte un codon stop et d'une délétion des exons 75 à 76 entraînant un décalage du cadre de lecture et de ce fait l'apparition d'un codon stop précoce activant le NMD sur cet ARNm, ont été utilisées.

Ces deux lignées cellulaires ont été traitées pendant 48 heures avec le composé 70, 71 ou 72, ou en présence de DMSO. Les ARN ont ensuite été extraits et une RT-PCR a été réalisée en condition quantitative.

Les résultats ont montré une stabilisation de l'ARNm dystrophine d'un facteur 4 environ, suite au traitement avec les composés 70, 71 ou 72.

### II-9 Inhibition du NMD in vivo:

Deux modèles murins pour le mécanisme de NMD ont été utilisés pour étudier l'effet *in vivo* du composé 70. Le premier modèle est appelé souris mdx. Ces souris portent une mutation non sens dans l'exon 23 du gène de la dystrophine. Ce codon non sens induit la dégradation de l'ARNm dystrophine par le NMD. Ces souris ont reçu dans le muscle une injection de DMSO (50 (µl), 20, 200 ou 2000 nmol de composé 70. Après 6, 24 et 32 heures, des souris ont été sacrifiées, le muscle injecté a été prélevé afin d'y extraire les ARN et les protéines. Les résultats préliminaires montrent une stabilisation de l'ARNm dystrophine dans les souris injectées par le composé 70 et non dans les souris injectées par le DMSO. De plus, la protéine dystrophine tronquée est elle aussi détectée dans ces souris injectées par le composé 70 et non dans les souris qui ont reçu le DMSO.

Le deuxième modèle murin qui a été utilisé se définit par la présence d'une mutation non sens dans l'exon 3 du gène µ du récepteur aux opiacés (MOR). Ce codon stop active le NMD sur cet ARNm MOR. Le gène MOR est exprimé seulement dans le système nerveux central ce qui fait de ce modèle un outil très intéressant afin d'étudier la possibilité pour un composé chimique de traverser la barrière hémato-encéphalique. La même procédure que pour les souris mdx a été suivie sauf que l'injection s'est faite en sous cutanée au niveau du cou des souris et le cerveau a été prélevé aux différents temps de l'expérience. Là encore, les premiers résultats montrent une stabilisation de l'ARNm MOR dans les souris injectées par le composé 70 et non dans les souris injectées par le DMSO seul. L'analyse par western-blot de la protéine MOR montre la présence de la protéine tronquée dans ces mêmes souris injectées par le composé 70 et non dans les souris n'ayant reçu que du DMSO. Ces résultats montrent que le composé 70 est actif *in vivo* et qu'il peut traverser la barrière hémato-encéphalique ce qui fait de ce composé un potentiel agent thérapeutique pour des maladies affectant le système nerveux central et liées au NMD.

### III- Discussion

Cette étude a permis de mettre en évidence la capacité d'inhibition du NMD de dérivés indoles. Ces composés agissent en empêchant l'interaction entre hUPF1 et hSMG5 ce qui aboutit à l'exclusion de hSMG5 des P-bodies et à la stabilisation des formes hyperphoshorylées de hUPF1.

Finalement, les résultats ont permis de mettre en évidence que ces dérivés indoles sont capables d'inhiber le NMD *in vivo* et de passer la barrière hématoencéphallique ce qui en fait de bons candidats dans le traitement de nombreuses pathologies associées au NMD.

Ces composés, en outre, s'avèrent être d'excellents outils pour étudier le mécanisme de NMD par une approche qui n'a jamais été possible du fait du manque d'outil pour inhiber spécifiquement ce processus. Un intérêt pour les laboratoires travaillant sur ce mécanisme de NMD est donc tout à fait concevable.

### Références :

Balkan, F. ; Elmes, B.C. ; Loder, J. W. Australian Journal of Chemistry 1969, 22, 2489.
Bashkirov, V.I., H. Scherthan, J.A. Solinger, J.M. Buerstedde, and W.D. Heyer. 1997. A mouse cytoplasmic exoribonuclease (mXRN1p) with preference for G4 tetraplex substrates. J Cell Biol. 136:761-73.
Belgrader, P., and L.E. Maquat. 1994. Nonsense but not missense mutations can decrease the abundance of nuclear mRNA for the mouse major urinary protein, while both types of mutations can facilitate exon skipping. Mol Cell Biol. 14:6326-36.
Brengues, M., D. Teixeira, and R. Parker. 2005. Movement of eukaryotic mRNAs between polysomes and cytoplasmic processing bodies. Science. 310:486-9.
Bisagni, E., Rautureau, M., Huel, C. Heterocycles 1988, 27, 1671-1678
Chen, C.Y., and A.B. Shyu. 2003. Rapid deadenylation triggered by a nonsense codon precedes decay of the RNA body in a mammalian cytoplasmic nonsense-mediated decay pathway. Mol Cell Biol. 23:4805-13.
Chiu, S.Y., G. Serin, O. Ohara, and L.E. Maquat. 2003. Characterization of human Smg5/7a: a protein with similarities to Caenorhabditis elegans SMG5 and SMG7 that functions in the dephosphorylation of Upfl . Rna. 9:77-87.
Conti, E., and E. Izaurralde. 2005. Nonsense-mediated mRNA decay: molecular insights and mechanistic variations across species. Curr Opin Cell Biol. 17:316-25.
Cougot, N., S. Babajko, and B. Seraphin. 2004. Cytoplasmic foci are sites of mRNA decay in human cells. J Cell Biol. 165:31-40.
Couttet, P., and T. Grange. 2004. Premature termination codons enhance mRNA decapping in human cells. Nucleic Acids Res. 32:488-94.
Czaplinski, K., M.J. Ruiz-Echevarria, S.V. Paushkin, X. Han, Y. Weng, H.A. Perlick, H.C. Dietz, M.D. Ter-Avanesyan, and S.W. Peltz. 1998. The surveillance complex interacts with the translation release factors to enhance termination and degrade aberrant mRNAs. Genes Dev. 12:1665-77.
Ducrocq, C., Bisagni, E. , Rivalle. C., Lhoste, J.M. Tetahedron 1979, 35, 142
Fenger-Gron, M., C. Fillman, B. Norrild, and J. Lykke-Andersen. 2005. Multiple processing body factors and the ARE binding protein TTP activate mRNA decapping. Mol Cell. 20:905-15.
Fukuhara, N., J. Ebert, L. Unterholzner, D. Lindner, E. Izaurralde, and E. Conti. 2005. SMG7 is a 14-3-3-like adaptor in the nonsense-mediated mRNA decay pathway. Mol Cell. 17:537-47.
Fusco, D., N. Accornero, B. Lavoie, S.M. Shenoy, J.M. Blanchard, R.H. Singer, and E. Bertrand. 2003. Single mRNA molecules demonstrate probabilistic movement in living mammalian cells. Curr Biol. 13:161-7.
Frischmeyer, P.A. and Dietz, H.C. 1999. Nonsense-mediated mRNA decay in health and disease. Human Molecular Genetics, 8(10):1893-1900.
Gehring, N.H., J.B. Kunz, G. Neu-Yilik, S. Breit, M.H. Viegas, M.W. Hentze, and A.E. Kulozik. 2005. Exon-junction complex components specify distinct routes of nonsense-mediated mRNA decay with differential cofactor requirements. Mol Cell. 20:65-75.
He, F., X. Li, P. Spatrick, R. Casillo, S. Dong, and A. Jacobson. 2003. Genome-wide analysis of mRNAs regulated by the nonsense-mediated and 5' to 3' mRNA decay pathways in yeast. Mol Cell. 12:1439-52.
Hosoda, N., Y.K. Kim, F. Lejeune, and L.E. Maquat. 2005. CBP80 promotes interaction of Upf1 with Upf2 during nonsense-mediated mRNA decay in mammalian cells. Nat Struct Mol Biol. 12:893-901.
Ingelfinger, D., D.J. Arndt-Jovin, R. Luhrmann, and T. Achsel. 2002. The human LSm1-7 proteins colocalize with the mRNA-degrading enzymes Dcp1/2 and Xrnl in distinct cytoplasmic foci. Rna. 8:1489-501.
Ishigaki, Y., X. Li, G. Serin, and L.E. Maquat. 2001. Evidence for a pioneer round of mRNA translation: mRNAs subject to nonsense-mediated decay in mammalian cells are bound by CBP80 and CBP20. Cell. 106:607-17.
Kashima, I., A. Yamashita, N. Izumi, N. Kataoka, R. Morishita, S. Hoshino, M. Ohno, G. Dreyfuss, and S. Ohno. 2006. Binding of a novel SMG-1-Upfl-eRF1-eRF3 complex (SURF) to the exon junction complex triggers Upf1 phosphorylation and nonsense-mediated mRNA decay. Genes Dev. 20:355-67.
Kedersha, N., G. Stoecklin, M. Ayodele, P. Yacono, J. Lykke-Andersen, M.J. Fitzler, D. Scheuner, R.J. Kaufman, D.E. Golan, and P. Anderson. 2005. Stress granules and processing bodies are dynamically linked sites of mRNP remodeling. J Cell Biol. 169:871-84.
Kim, Y.K., L. Furic, L. Desgroseillers, and L.E. Maquat. 2005. Mammalian Staufen1 recruits Upf1 to specific mRNA 3'UTRs so as to elicit mRNA decay. Cell. 120:195-208.
Kuzmiak, H.A., and L.E. Maquat. 2006. Applying nonsense-mediated mRNA decay research to the clinic: progress and challenges. Trends Mol Med*.*
Lejeune, F., X. Li, and L.E. Maquat. 2003. Nonsense-mediated mRNA decay in mammalian cells involves decapping, deadenylating, and exonucleolytic activities. Mol Cell. 12:675-87.
Lejeune, F., and L.E. Maquat. 2004. Immunopurification and analysis of protein and RNA components of mRNP in mammalian cells. Methods Mol Biol. 257:115-24.
Lejeune, F., and L.E. Maquat. 2005. Mechanistic links between nonsense-mediated mRNA decay and pre-mRNA splicing in mammalian cells. Curr Opin Cell Biol. 17:309-15.
Lykke-Andersen, J., M.D. Shu, and J.A. Steitz. 2000. Human Upf proteins target an mRNA for nonsense-mediated decay when bound downstream of a termination codon. Cell. 103:1121-31.
Maquat, L.E. 2004a. Nonsense-Mediated mRNA Decay: A Comparative Analysis of Different Species. Current Genomics. 5:175-190.
Maquat, L.E. 2004b. Nonsense-mediated mRNA decay: splicing, translation and mRNP dynamics. Nat Rev Mol Cell Biol. 5:89-99.
Mendell, J.T., C.M. ap Rhys, and H.C. Dietz. 2002. Separable roles for rentl/hUpfl in altered splicing and decay of nonsense transcripts. Science. 298:419-22.
Mendell, J.T., N.A. Sharifi, J.L. Meyers, F. Martinez-Murillo, and H.C. Dietz. 2004. Nonsense surveillance regulates expression of diverse classes of mammalian transcripts and mutes genomic noise. Nat Genet. 36:1073-8.
Moriarty, P.M., C.C. Reddy, and L.E. Maquat. 1998. Sélénium deficiency reduces the abundance of mRNA for Se-dependent glutathione peroxidase 1 by a UGA-dependent mechanism likely to be nonsense codon-mediated decay of cytoplasmic mRNA. Mol Cell Biol. 18:2932-9.
Ohnishi, T., A. Yamashita, I. Kashima, T. Schell, K.R. Anders, A. Grimson, T. Hachiya, M.W. Hentze, P. Anderson, and S. Ohno. 2003. Phosphorylation of hUPF1 induces formation of mRNA surveillance complexes containing hSMG-5 and hSMG-7. Mol Cell. 12:1187-200.
Page, M.F., B. Carr, K.R. Anders, A. Grimson, and P. Anderson. 1999. SMG-2 is a phosphorylated protein required for mRNA surveillance in Caenorhabditis elegans and related to Upflp of yeast. Mol Cell Biol. 19:5943-51.
Pal, M., Y. Ishigaki, E. Nagy, and L.E. Maquat. 2001. Evidence that phosphorylation of human Upf1 protein varies with intracellular location and is mediated by a wortmannin-sensitive and rapamycin-sensitive PI 3-kinase-related kinase signaling pathway. Rna. 7:5-15.
Pillai, R.S., S.N. Bhattacharyya, C.G. Artus, T. Zoller, N. Cougot, E. Basyuk, E. Bertrand, and W. Filipowicz. 2005. Inhibition of translational initiation by Let-7 MicroRNA in human cells. Science. 309:1573-6.
Rehwinkel, J., I. Letunic, J. Raes, P. Bork, and E. Izaurralde. 2005. Nonsense-mediated mRNA decay factors act in concert to regulate common mRNA targets. Rna. 11:1530-44.
Rivalle, C., Ducrocq, C., Lhoste, J. M., Wendling, F.,Bisagnie, E., Chermann, J. C. Tetrahedron 1981 ; 37, 2097-2103
Rivalle, C. , Wendling, F. , Tambourin, P. , Lhoste, J. M. , Bisagni, E. J. Med. Chem. 1983, 26, 181-185Serin, G., A. Gersappe, J.D. Black, R. Aronoff, and L.E. Maquat. 2001. Identification and characterization of human orthologues to Saccharomyces cerevisiae Upf2 protein and Upf3 protein (Caenorhabditis elegans SMG-4). Mol Cell Biol. 21:209-23.
Sheth, U., and R. Parker. 2003. Decapping and decay of messenger RNA occur in cytoplasmic processing bodies. Science. 300:805-8.
Sheth, U., and R. Parker. 2006. Targeting of aberrant mRNAs to cytoplasmic processing bodies. Cell. 125:1095-109.
Soret, J., N. Bakkour, S. Maire, S. Durand, L. Zekri, M. Gabut, W. Fic, G. Divita, C. Rivalle, D. Dauzonne, C.H. Nguyen, P. Jeanteur, and J. Tazi. 2005. Selective modification of alternative splicing by indole derivatives that target serine-arginine-rich protein splicing factors. Proc Natl Acad Sci U S A. 102:8764-9.
Sun, X., H.A. Perlick, H.C. Dietz, and L.E. Maquat. 1998. A mutated human homologue to yeast Upf1 protein has a dominant-negative effect on the decay of nonsense-containing mRNAs in mammalian cells. Ploc Natl Acad Sci U S A. 95 :10009-14.
Sureau, A., R. Gattoni, Y. Dooghe, J. Stevenin, and J. Soret. 2001. SC35 autoregulates its expression by promoting splicing events that destabilize its mRNAs. Embo J. 20:1785-96.
Teixeira, D., U. Sheth, M.A. Valencia-Sanchez, M. Brengues, and R. Parker. 2005. Processing bodies require RNA for assembly and contain nontranslating mRNAs. Rna. 11:371-82.
Thermann, R., G. Neu-Yilik, A. Deters, U. Frede, K. Wehr, C. Hagemeier, M.W. Hentze, and A.E. Kulozik. 1998. Binary spécification of nonsense codons by splicing and cytoplasmic translation. Embo J. 17:3484-94.
Tourriere, H., K. Chebli, L. Zekri, B. Courselaud, J.M. Blanchard, E. Bertrand, and J. Tazi. 2003. The RasGAP-associated endoribonuclease G3BP assembles stress granules. J Cell Biol. 160:823-31.
Unterholzner, L., and E. Izaurralde. 2004. SMG7 acts as a molecular link between mRNA surveillance and mRNA decay. Mol Cell. 16:587-96.
van Dijk, E., N. Cougot, S. Meyer, S. Babajko, E. Wahle, and B. Seraphin. 2002. Human Dcp2: a catalytically active mRNA decapping enzyme located in specific cytoplasmic structures. Embo J. 21:6915-24.
Wollerton, M.C., C. Gooding, E.J. Wagner, M.A. Garcia-Blanco, and C.W. Smith. 2004. Autoregulation of polypyrimidine tract binding protein by alternative splicing leading to nonsense-mediated decay. Mol Cell. 13:91-100.
Yamashita, A., T. Ohnishi, I. Kashima, Y. Taya, and S. Ohno. 2001. Human SMG-1, a novel phosphatidylinositol 3-kinase-related protein kinase, associates with components of the mRNA surveillance complex and is involved in the régulation of nonsense-mediated mRNA decay. Genes Dev. 15:2215-28.
Yu, J.H., W.H. Yang, T. Gulick, K.D. Bloch, and D.B. Bloch. 2005. Ge-1 is a central component of the mammalian cytoplasmic mRNA processing body. Rna. 11:1795-802.
Zhang, J., X. Sun, Y. Qian, and L.E. Maquat. 1998. Intron function in the nonsense-mediated decay of beta-globin mRNA: indications that pre-mRNA splicing in the nucleus can influence mRNA translation in the cytoplasm. Rna. 4:801-15.

## Revendications

1. Un composé dérivé d'indole correspondant à la formule II suivante : 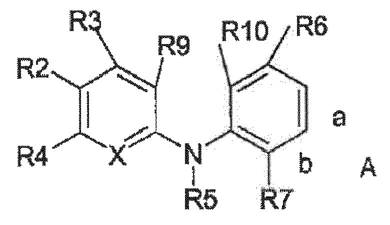 dans laquelle :
- X représente N ou l'anhydro base N⁺R8,
où R8 représente un atome d'hydrogène, un groupement hydroxyle ou alkyle ou méthoxy éventuellement substitué par un groupement phényle, de préférence R8 représente un atome d'hydrogène,
- R2, R3 et R4 représentent indépendamment un atome d'hydrogène ou un atome d'halogène ou un groupement alkyle en C1-C4,
- R5 représente un atome d'hydrogène ou un groupement alkyle saturé ou insaturé,
- R6 représente un groupement alkyle en C1-C3, de préférence un groupement méthyle ou éthyle et de manière particulièrement préférée R6 représente un groupement méthyle,
- R7 représente un atome d'hydrogène ou un groupement alkyle en C1-C3,
- R9, R10 représentent indépendamment un atome d'hydrogène, un groupement R11, OR11, ou SR11,
où R11 représente un atome d'hydrogène, un atome d'oxygène, un groupement alkyle en C1-C3 saturé ou insaturé, pouvant contenir un ou plusieurs atomes de soufre, d'oxygène ou d'azote ;
- A représente un cycle en position a et qui correspond à 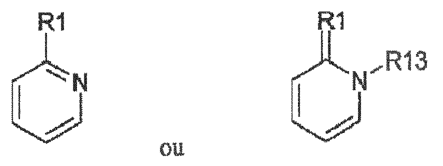 dans lequel :
- R1 représente un atome d'hydrogène, d'oxygène ou d'halogène ou un groupement alkyle ou amine linéaire ou ramifié et/ou insaturé,
- R13 représente un atome d'hydrogène ou un groupement alkyle en C1-C4, et/ou des sels pharmaceutiquement acceptables desdits composés.

2. Un composé dérivé d'indole selon la revendication 1, de formule Ia suivante 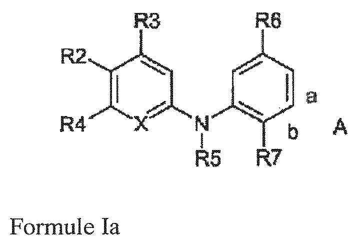 dans laquelle R2, R3, R4, R5, R6, R7, X et le cycle A sont tels que définis dans la revendication1.

3. Un composé selon l'une quelconque des revendications précédentes, **caractérisé en ce que** R2, R3 et R4 représentent indépendamment les uns des autres un atome d'hydrogène ou un atome d'halogène.

4. Un composé selon l'une quelconque des revendications précédentes, caractérisé en ce R5 représente un groupement méthyle, éthyle, propyle ou butyle et, de préférence, R5 représente un atome d'hydrogène.

5. Un composé selon l'une quelconque des revendications précédentes, **caractérisé en ce que** R7 représente un groupement méthyle ou éthyle et, de préférence, R7 représente un groupement méthyle.

6. Un composé selon l'une quelconque des revendications précédentes, **caractérisé en ce que** R1 est un atome d'hydrogène, d'oxygène ou d'halogène.

7. Un composé selon l'une quelconque des revendications précédentes, **caractérisé en ce que** A est choisi dans le groupe comprenant :

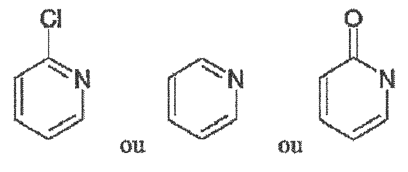

8. Un composé selon l'une quelconque des revendications précédentes, **caractérisé en ce que** le composé est choisi dans le groupe comprenant :
- 5,8-Dimethyl-6-(pyridin-2-ylamino)-2H-isoquinolin-1-one;
- 5,8-diméthyl -6-(3 méthoxy-pyridin-2-ylamino)-isoquinolin-1-one; et
- 5,8-diméthyl-6-(5-méthyl-pyridin-2-ylamino)-isoquinoline, et, de préférence ledit composé est le 5, 8-Dimethyl-6-(pyridin-2-ylamino)-2H-isoquinolin-1-one.

9. Composition pharmaceutique comprenant au moins un dérivé d'indole tel que défini dans l'une quelconque des revendications 1 à 8 et, éventuellement, un support pharmaceutiquement acceptable.

10. Composé dérivé d'indole correspondant à la formule II suivante : 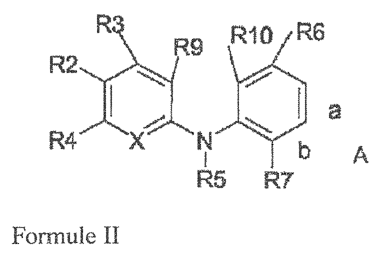 dans laquelle :
- X représente N, CR8 ou l'anhydro base N⁺R8,
où R8 représente un atome d'hydrogène, un groupement hydroxyle ou alkyle ou méthoxy éventuellement substitué par un groupement phényle, de préférence R8 représente un atome d'hydrogène,
- R2, R3 et R4 représentent indépendamment un atome d'hydrogène ou un atome d'halogène ou un groupement alkyle en C1-C4,
- R5 représente un atome d'hydrogène ou un groupement alkyle, saturé ou insaturé,
- R6 représente un groupement alkyle en C1-C3, de préférence un groupement méthyle ou éthyle et de manière particulièrement préférée R6 représente un groupement méthyle,
- R7 représente un atome d'hydrogène ou un groupement alkyle en C1-C3
- R9 et R10 représentent ensemble une liaison carbone ou représentent indépendamment un atome d'hydrogène, un groupement R11, OR11, ou SR11,
où R11 représente un atome d'hydrogène, un atome d'oxygène, un groupement alkyle en C1-C3 saturé ou insaturé, pouvant contenir un ou plusieurs atomes de soufre, d'oxygène ou d'azote,
- A représente un cycle en position a et qui correspond à 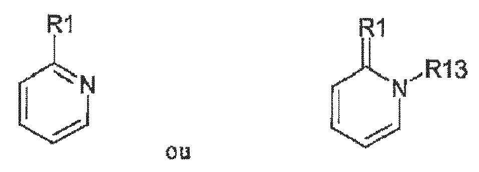 dans lequel :
- R1 représente un atome d'hydrogène, d'oxygène ou d'halogène ou un groupement alkyle ou amine linéaire ou ramifié et/ou insaturé,
- R13 représente un atome d'hydrogène ou un groupement alkyle en C1-C4, et/ou des sels pharmaceutiquement acceptables desdits composés,
pour son utilisation pour traiter, chez un sujet, une maladie génétique choisie dans le groupe comprenant la β-thalassémie, le syndrome de Marfan, la dystrophie musculaire de Duchenne (DMD), la dystrophie musculaire de Becker (BMD), la maladie d'Ullrich, le syndrome de Hurler et la fibrose cystique (CF).

11. Utilisation d'un composé selon la revendication 10, **caractérisée en ce que** ledit composé est choisi dans le groupe comprenant :
- 6-Chloro-5, 10-dimethyt-11H-pyrido[3',2':4,5]pyrrolo[3,2-g]isoquinoline ;
- 5,10-dimethyl-11H-pyrido[3',2':4,5]pyrrolo[3,2-g]isoquinoline ;
- 5,8-Dimethyl-6-(pyridin-2-ylamino)-2H-isoquinolin-1-one;
- 5,8-Dimethyl 6-(3 méthoxy-pyridin-2-ylamino)-isoquinolin-1-one ; et
- 5,8-diméthyl-6-(5-méthyl-pyridin-2-ylamino)-isoquinoline.

## Patentansprüche

1. Indolderivatverbindung der folgenden Formel II: 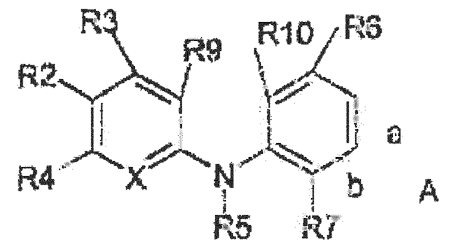 worin:
- X N oder die Anhydrobase N⁺R8 darstellt,
worin R8 ein Wasserstoffatom, eine Hydroxy- oder Alkyl- oder Methoxygruppe darstellt, die gegebenenfalls mit einer Phenylgruppe substituiert ist, wobei R8 vorzugsweise ein Wasserstoffatom darstellt,
- R2, R3 und R4 unabhängig voneinander ein Wasserstoffatom oder ein Halogenatom oder eine Cl-C4-Alkylgruppe darstellen,
- R5 ein Wasserstoffatom oder eine gesättigte oder ungesättigte Alkylgruppe darstellt,
- R6 eine C1-C3-Alkylgruppe darstellt, wobei eine Methyl- oder Ethylgruppe bevorzugt ist, wobei eine Methylgruppe besonders bevorzugt ist,
- R7 ein Wasserstoffatom oder eine C1-C3-Alkylgruppe darstellt,
- R9, R10 unabhängig voneinander ein Wasserstoffatom, eine Gruppe R11, OR11 oder SR11 darstellen,
worin R11 ein Wasserstoffatom, ein Sauerstoffatom oder eine gesättigte oder ungesättigte C1-C3-Alkylgruppe darstellt, die ein oder mehrere Schwefel-, Sauerstoff- oder Stickstoffatome enthalten kann;
- A einen Ring an der Position a darstellt und 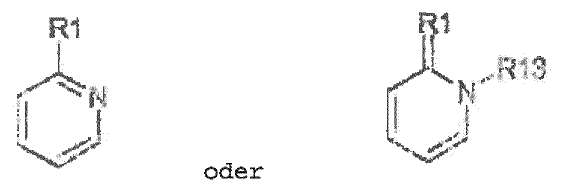
entspricht, worin:
- R1 ein Wasserstoffatom, Sauerstoffatom oder Halogenatom oder eine Alkyl- oder Amingruppe darstellt, die linear oder verzweigt und/oder ungesättigt sein kann,
- R13 ein Wasserstoffatom oder eine C1-C4-Alkylgruppe darstellt,
und/oder pharmazeutisch geeignete Salze davon.

2. Indolderivatverbindung nach Anspruch 1 der Formel Ia 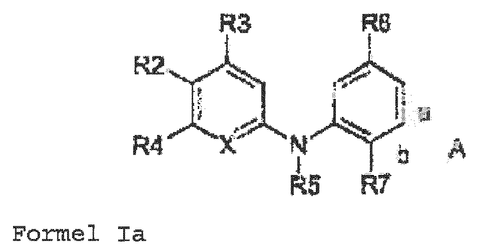 worin R2, R3, R4, R5, R6, R7, X und der Ring A dieselbe Bedeutung wie im Anspruch 1 haben.

3. Verbindung nach einem der vorstehenden Ansprüche **dadurch gekennzeichnet, dass** R2, R3 und R4 unabhängig voneinander ein Wasserstoffatom oder ein Halogenatom darstellen.

4. Verbindung nach einem der vorstehenden Ansprüche, **dadurch gekennzeichnet, dass** R5 eine Methyl-, Ethyl-, Propyl- oder Butylgruppe darstellt und R5 vorzugsweise ein Wasserstoffatom ist.

5. Verbindung nach einem der vorstehenden Ansprüche, **dadurch gekennzeichnet, dass** R7 eine Methyl- oder Ethylgruppe ist und vorzugsweise eine Methylgruppe ist.

6. Verbindung nach einem der vorstehenden Ansprüche, **dadurch gekennzeichnet, dass** R1 ein Wasserstoffatom, Sauerstoffatom oder Halogenatom ist.

7. Verbindung nach einem der vorstehenden Ansprüche, **dadurch gekennzeichnet, dass** A aus der folgenden Gruppe ausgewählt ist:

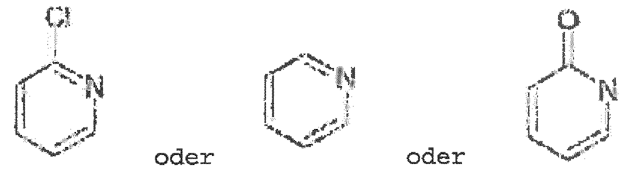

8. Verbindung nach einem der vorstehenden Ansprüche, **dadurch gekennzeichnet, dass** die Verbindung aus der folgenden Gruppe ausgewählt ist:
- 5,8-Dimethyl-6-(pyridin-2-ylamino)-2H-isochinolin-1-on;
- 5,8-Dimehtyl-6-(3-methoxypyridin-2-ylamino)isochinolin-1-on; und
- 5,8-Dimethyl-6-(5-methylpyridin-2-ylamino)isochinolin, wobei die Verbindung vorzugsweise 5,8-Dimethyl-6-(pyridin-2-ylamino)-2H-isochinolin-1-on ist.

9. Arzneimittelzusammensetzung, die ein Indolderivat nach einem der Ansprüche 1 bis 8 und gegebenenfalls einen geeigneten Arzneimittelträger enthält.

10. Indolderivatverbindung der folgenden Formel II: 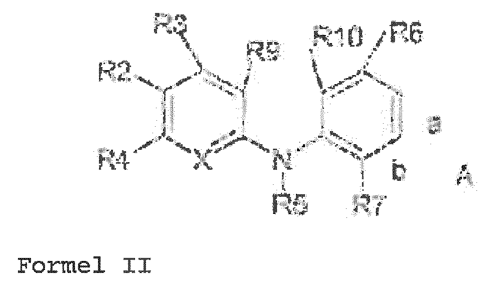 worin:
- X N, CR8 oder die Anhydrobase N⁺R8 darstellt, worin R8 ein Wasserstoffatom, eine Hydroxy- oder Alkyl- oder Methoxygruppe darstellt, die gegebenenfalls mit einer Phenylgruppe substituiert ist, wobei R8 vorzugsweise ein Wasserstoffatom darstellt,
- R2, R3 und R4 unabhängig voneinander ein Wasserstoffatom oder ein Halogenatom oder eine C1-C4-Alkylgruppe darstellen,
- R5 ein Wasserstoffatom oder eine gesättigte oder ungesättigte Alkylgruppe darstellt,
- R6 eine C1-C3-Alkylgruppe darstellt, vorzugsweise eine Methyl- oder Ethylgruppe und stärker bevorzugt eine Methylgruppe,
- R7 ein Wasserstoffatom oder eine C1-C3-Alkylgruppe darstellt,
- R9 und R10 zusammen eine Kohlenstoffverbindung darstellen oder unabhängig voneinander ein Wasserstoffatom oder eine Gruppe R11, OR11 oder SR11 darstellen,
worin R11 ein Wasserstoffatom, ein Sauerstoffatom oder eine gesättigte oder ungesättigte C1-C3-Alkylgruppe darstellt, die ein oder mehrere Schwefel-, Sauerstoff- oder Stickstoffatome enthalten kann,
- A einen Ring in der Position a darstellt und 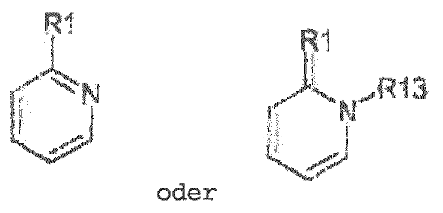
entspricht, worin:
- R1 ein Wasserstoff-, Sauerstoff- oder Halogenatom oder eine Alkyl- oder Amingruppe darstellt, die linear oder verzweigt und/oder ungesättigt ist,
- R13 ein Wasserstoffatom oder eine C1-C4-Alkylgruppe darstellt,
und/oder pharmazeutisch geeignete Salze davon,
für die Verwendung zur Behandlung einer Person mit einer genetischen Erkrankung, die aus der Gruppe ausgewählt ist, die aus 1a β-Thalassämie, dem Marfan-Syndrom, Duchenne-Muskeldystrophie (DMD), Becker-Muskeldystrophie (BMD), der Ullrichkrankheit, dem Hurler-Syndrom und zystischer Fibrose (CF) besteht.

11. Verwendung einer Verbindung nach Anspruch 10, **dadurch gekennzeichnet, dass** die Verbindung aus der folgenden Gruppe ausgewählt ist:
- 6-Chloro-5,10-dimethyl-11H-pyrido[3',2':4,5]pyrrolo[3,2-g]isochinolin;
- 5,10-Dimethyl-11H-pyrido[3',2':4,5]pyrrolo[3,2-g]isochino-lin;
- 5,8-Dimethyl-6-(pyridin-2-ylamino)-2H-isochinolin-1-on;
- 5,8-Dimehtyl-6-(3-methoxy-pyridin-2-ylamino)isochinolin-1-on; und
- 5,8-Dimehtyl-6-(5-methyl-pyridin-2-ylamino)isochinolin.

## Claims

1. A compound derived from indole corresponding to the following formula II: 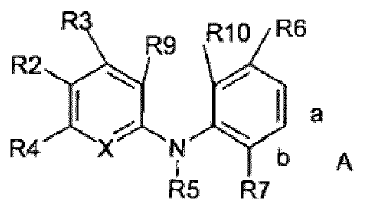 wherein:
- X represents N or the anhydrobase N⁺R8, wherein R8 represents a hydrogen atom, a hydroxyl or alkyl or methoxy group optionally substituted with a phenyl group, preferably R8 represents a hydrogen atom,
- R2, R3 and R4 independently represent a hydrogen atom or a halogen atom or a C₁-C₄ alkyl group,
- R5 represents a hydrogen atom or an saturated or unsaturated alkyl group,
- R6 represents an optionally substituted C₁-C₃ alkyl group, preferably a methyl or ethyl group, and more preferably R6 represents a methyl group,
- R7 represents a hydrogen atom or a C₁-C₃ alkyl group,
- R9, R10 independently represent a hydrogen atom, a R11, OR11 or SR11 group,
wherein R11 represents a hydrogen atom, an oxygen atom, a saturated or unsaturated, C₁-C₃ alkyl group, which may contain one or more sulfur, oxygen or nitrogen atoms;
- A represents a ring in the a position, and which corresponds to 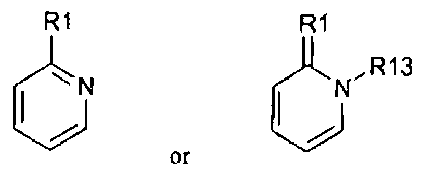
wherein:
- R1 represents a hydrogen, oxygen or halogen atom, or a, linear or branched and/or unsaturated alkyl or amine group,
- R13 represents a hydrogen atom or C₁-C₄ alkyl group and/or
pharmaceutically acceptable salts of said compounds.

2. The compound derived from indole according to claim 1, of the following formula 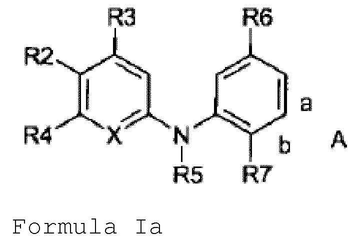 wherein R2, R3, R4, R5, R6, R7, X and the ring A are as defined in claim 1.

3. A compound according to any of the preceding claims, **characterized in that** R2, R3 and R4 represent independently of each other, a hydrogen atom or a halogen atom.

4. A compound according to any of the preceding claims, **characterized in that** this R5 represents a methyl, ethyl, propyl or butyl group and preferably R5 represents a hydrogen atom.

5. A compound according to any of the preceding claims, **characterized in that** R7 represents a methyl or ethyl group and preferably R7 represents a methyl group.

6. A compound according to any of the preceding claims, **characterized in that** R1 is a hydrogen, oxygen or halogen atom.

7. A compound according to any of the preceding claims, **characterized in that** A is selected from the group comprising:

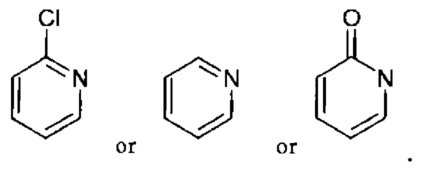

8. A compound according to any of the preceding claims, **characterized in that** the compound is selected from the group comprising:
• 5,8-dimethyl-6-(pyridin-2-ylamino)-2*H-*isoquinolin-1-one;
• 5,8-dimethyl-6-(3-methoxy-pyridin-2-ylamino)-isoquinolin-1-one; and
• 5,8-dimethyl-6(5-methyl-pyridin-2-ylamino)-isoquinoline, and preferably said compound is 5,8-dimethyl-6-(pyridin-2-ylamino)-2H-isoquinolin-1-one.

9. A pharmaceutical composition comprising at least an indole derivative as defined according to any of claims 1 to 8, and optionally a pharmaceutically acceptable support.

10. A compound derived from indole corresponding to the following formula II: 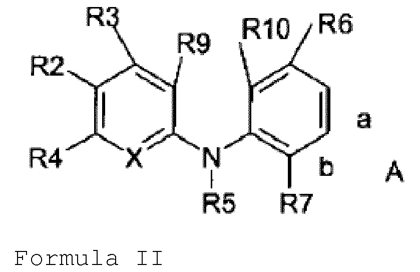 wherein:
- X represents N, CR8 or the anhydrobase N⁺R8,
wherein R8 represents a hydrogen atom, a hydroxyl or alkyl or methoxy group optionally substituted with a phenyl group, preferably R8 represents a hydrogen atom,
- R2, R3 and R4 independently represent a hydrogen atom or a halogen atom or a C₁-C₄ alkyl group,
- R5 represents a hydrogen atom or a saturated or unsaturated alkyl group,
- R6 represents a C₁-C₃ alkyl group, preferably a methyl or ethyl group, and more preferably R6 represents a methyl group,
- R7 represents a hydrogen atom or a C₁-C₃ alkyl group,
- R9 and R10 represent together a carbon bond or independently represent a hydrogen atom, a R11,
OR11, SR11 group,
wherein R11 represents a hydrogen atom, an oxygen atom, a saturated or unsaturated, C₁-C₃ alkyl group, which may contain one or more sulfur, oxygen or nitrogen atoms,
- A represents a ring in position a and which corresponds to: 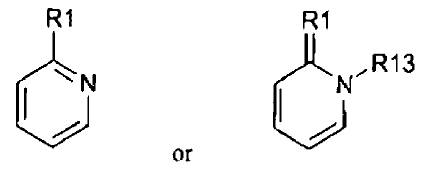
wherein:
- R1 represents a hydrogen, oxygen or halogen atom or a linear or branched and/or unsaturated alkyl or amine group,
- R13 represents a hydrogen atom or a C₁-C₄ alkyl group, and/or pharmaceutically acceptable salts of said compounds
for its use for treating, in a subject, a genetic disease selected from the group comprising β-thalassemia, Marfan's syndrome, Duchenne's muscular dystrophy (DMD), Becker's muscular dystrophy (BMD), Ullrich's disease, Hurler's syndrome and cystic fibrosis (CF).

11. The use of a compound according to claim 10, **characterized in that** said compound is selected from the group comprising:
- 6-chloro-5,10-dimethyl-1 1H-pyrido[3',2':4,5]pyrrolo[3,2-g]isoquinoline:
- 5,10-dimethyl-1 1H-pyrido[3',2':4,5]pyrrolo[3,2-g]isoquinoline;
- 5,8-dimethyl-6-(pyridin-2-ylamino)-2H-isoquinolin-1-one;
- 5,8-dimethyl-6-(3-methoxy-pyridin-2-ylamino)-isoquinolin-l-one; and
- 5,8-dimethyl-6-(5-methyl-pyridin-2-ylamino)-isoquinoline.


### SEQUENCE LISTING

<110> CENTRE NATIONAL DE LA RECHERCHE SCIENTIFIQUE (CNRS) Institut Curie LEJEUNE, Fabrice TAZI, Jamal GRIERSON, David RIVALLE, Christian MAHUTEAU-BETZER, Florence
<120> NOUVEAUX COMPOSÉS DÉRIVÉS D'INDOLE ET COMPOSITIONS PHARMACEUTIQUES LES CONTENANT
<130> 352497
<150> FR07/53349
   <151> 2007-02-19
<150> FR07/53975
   <151> 2007-03-21
<160> 2
<170> PatentIn version 3.3
<210> 1
   <211> 33
   <212> DNA
   <213> Artificial
<220>
   <223> amorce PCR
<400> 1
   gcaacctcaa gcttacacca tggtgcacct gac 33
<210> 2
   <211> 30
   <212> DNA
   <213> Artificial
<220>
   <223> amorce PCR
<400> 2
   agaaagcaga tctgcttagt gatacttgtg 30